# EUROPEAN PATENT SPECIFICATION

(11) **EP 1 274 675 B1**
(45) Date of publication and mention of the grant of the patent: **01.09.2004**
(21) Application number: 00921148.3
(22) Date of filing: 19.04.2000
(51) Int. Cl.: C07C 229/64, C07C 311/21, A61K 31/196

(54) **COMPOUNDS, COMPOSITIONS AND METHODS FOR PREVENTING NEURODEGENERATION IN ACUTE AND CHRONIC INJURIES IN THE CENTRAL NERVOUS SYSTEM**
VERBINDUNGEN, ZUSAMMENSETZUNGEN UND VERFAHREN ZUR VORBEUGUNG DER NEURODEGENERATION BEI AKUTEN ODER CHRONISCHEN VERLETZUNGEN DES ZENTRALEN NERVENSYSTEMS
COMPOSES, COMPOSITIONS ET PROCEDES DE PREVENTION DE LA NEURODEGENERESCENCE DANS DES ATTEINTES LESIONNELLES AIGUES ET CHRONIQUES AU SYSTEME NERVEUX CENTRAL

(43) Date of publication of application: 15.01.2003
(73) Proprietor: Neurotech Co., Ltd., Suwon-si, Kyunggi-do 442-380 (KR)
(72) Inventor: GWAG, Byoung Joo, Seocho-ku, Seoul 137-063 (KR); LEE, Young Ae c/o Won Intern. Patent and Law Firm, 642-16 Yeoksam-dong, Gangnam-gu (KR); RYU, Bo Rum, Suwon -si, Kyunggi-do 442-371 (KR); YOON, Sung Hwa, Paldal-ku, Swon-si, Kyunggi-do 442-470 (KR); MOON, Ho Sang, Paldal-ku, Suwon-si, Kyunggi-do 442-190 (KR)
(74) Representative: Grünecker, Kinkeldey, Stockmair & Schwanhäusser Anwaltssozietät
(86) International application number: PCT/KR2000/000366
(87) International publication number: WO 2001/079153

(56) References cited:
- EP-A1- 0 122 827
- WO-A1-86/03199
- WO-A1-94/13663
- DE-A- 2 031 227
- CHEMICAL ABSTRACTS, vol. 122, no. 1, 02 January 1995, Columbus, Ohio, US; abstract no. 9738S, NUSSBAUMER P. ET AL.: 'Novel antiproliferative agents derived from lavendustin A' page 1068; column 1; XP002957043 & J. MED. CHEM. vol. 37, no. 24, 1994, pages 4079 - 4084
- CHEMICAL ABSTRACTS, vol. 67, no. 5, 31 July 1967, Columbus, Ohio, US; abstract no. 21558M, MYSYK D.D. ET AL.: 'Chloromethylation of 4-arenesulfonyl-N-methylamidoanisoles' page 2034; column 2; XP002957044 & UKR. KHIM. ZH. vol. 33, no. 2, 1967, pages 185 - 188
- PATENT ABSTRACTS OF JAPAN vol. 3, no. 147 (C-66) 05 December 1979 & JP 54 125 632 A (HISAMITSU SEIYAKU KK) 29 September 1979
- PATENT ABSTRACTS OF JAPAN vol. 10, no. 107 (C-341) 22 April 1986 & JP 60 237 041 A (YAMAMOTO KAGAYU KOGYO KK) 25 November 1985
- CHEMICAL ABSTRACTS, vol. 120, no. 15, 11 April 1994, Columbus, Ohio, US; abstract no. 190897Q, RIZZI E. ET AL.: 'Electron impact mass spectrometry of some 5-amin-osalicylic acid derivatives' page 972; column 1; XP002957045 & RAPID COMMUN. MASS SPECTROM. vol. 8, no. 2, 1994, pages 158 - 162

## Description

The present invention is related to novel salicylic compounds and compositions useful in prevention and prophylaxis of neurological diseases accompanied by neuronal death.

Excess activation of ionotropic glutamate receptors sensitive to N-methyl-D-aspartate (NMDA receptors) produces neuronal death and has been known to mediate various neurological diseases [Choi, Neuron 1:623-634 (1988)]. Glutamate, the excitatory neurotransmitter, is massively accumulated in brain subjected to hypoxic-ischemic injuries, which activates ionotropic glutamate receptors permeable to Ca²⁺ and Na⁺ and then causes neuronal death [Choi and Rothman, *Annu Rev Neurosci* 13:171-182 (1990)]. Antagonists of NMDA receptors remarkably attenuate brain injury following hypoglycemia, hypoxia, or hypoxic-ischemia [Simon, Swan, Griffiths, and Meldrum. *Science* 226:850-852 (1984); Park, Nehls, Graham, Teasdale, and McCulloch, *Ann Neurol* 24:543-551 (1988).;Wieloch, *Science* 230:681-683 (1985); Kass, Chambers, and Cottrell, *Exp.Neurol.* 103:116-122 (1989); Weiss, Goldberg, and Choi, *Brain Res.* 380:186-190 (1986)]. Thus, NMDA receptor antagonists possess therapeutic potentials to protect brain against hypoglycemia, hypoxia, and hypoxic-ischemic injuries.

Excitotoxicity appears to contribute to neuronal degeneration following traumatic brain injury (TBI). Levels of quinolinic acid, an endogenous agonist of NMDA receptors, was increased 5- to 50-fold in human patients with TBI [E. H. Sinz, P. M. Kochanek, M. P. Heyes, S. R. Wisniewski, M. J. Bell, R. S. Clark, S. T. DeKosky, A. R Blight, and D. W. Marion]. Quinolinic acid is increased in the cerebrospinal fluid and associated with mortality after TBI in humans [*J.Cereb.Blood Flow Metab.* 18:610-615, (1998)]. In animal models of brain trauma, levels of glutamate and aspartate were markedly increased [Faden, Demediuk, Panter, and Vink, *Science* 244:798-800 (1989)]. Glutamate release was also observed in rat spinal cord following impact trauma [Demediuk, Daly, and Faden. J Neurochem J. *Neurochem.* 52 :1529-1536 (1989)]. NMDA receptor antagonists attenuate neuronal death following traumatic brain or spinal cord injuries [Faden, Lemke, Simon, and Noble. *J.Neurotrauma.* 5:33-45(1988); Okiyama, Smith, White, Richter, and McIntosh. *J.Neurotrauma.* 14:211-222 (1997)].

Glutamate plays a central role in the induction and the propagation of seizures [Dingledine, McBain, and McNamara, *Trends.Pharmacol.Sci.* 11:334-338 (1990); Holmes. *Cleve.Clin.J.Med.* 62:240-247 (1995)]. NMDA receptor antagonists were shown to act as anticonvulsants and antiepileptogenic drugs in various models of epilepsy [Anderson, Swartzwelder, and Wilson, *J.Neurophysiol.* 57:1-21 (1987); Wong, Coulter, Choi, and Prince. *Neurosci.Lett.* 85:261-266 (1988); McNamara, Russell, Rigsbee, and Bonhaus, *Neuropharmacology* 27:563-568 (1988)].

Amyotrophic lateral sclerosis (ALS) is accompanied by degeneration of both upper and lower motor neurons and marked by neurogenic atrophy, weakness, and fasciculation. While the pathogenesis of ALS remains to be resolved, excitotoxicity has been expected to participate in the process of ALS. In particular, ALS patients show increased levels of extracellular glutamate and defects in glutamate transport. Administration of excitotoxins mimicked pathological changes in the spinal cord of ALS patients [Rothstein. *Clin.Neurosci.* 3:348-359 (1995); Ikonomidou, Qin, Labruyere, and Olney *J.Neuropathol.Exp.Neurol.* 55:211-224 (1996)].

Antagonizing NMDA receptors appears to be applied to treat Parkinson's disease (PD). Several antagonists of NMDA receptors protect dopaminergic neurons from the neurotoxin MPTP (1-methyl-4-phenyl-1,2,3,6-tetrahydropyridine) [Lange, Loschmann, Sofic, Burg, Horowski, Kalveram, Wachtel, and Riederer. *Naunyn Schmiedebergs Arch.Pharmacol.* 348:586-592 (1993); Brouillet and Beal. *Neuroreport.* 4:387-390 (1993)]. NMDA receptor antagonists also ameliorate levodopa-induced dyskinesia and thus can improve the therapeutic effects of levodopa [Papa and Chase, *Ann.Neurol.* 39:574-578 (1996); Marin, Papa, Engber, Bonastre, Tolosa, and Chase, *Brain Res.* 736:202-205 (1996)]. Two NMDA receptor antagonists, memantine and dextromethophan, have been proved beneficial in treating PD patients [Verhagen, Del Dotto, Natte, van den Munckhof, and Chase, *Neurology* 51:203-206 (1998); Merello, Nouzeilles, Cammarota, and Leiguarda. *Clin.Neuropharmacol.* 22:273-276 (1999)].

Huntington's disease (HD) is a progressive neurodegenerative disease predominantly affecting small- and medium-sized interneurons but sparing NADPH-diaphorase neurons containing somatostatin and neuropeptide in the striata. These pathological features of HD are observed in the striatal tissues following the intrastriatal injections of quinolinic acid or cultured striatal neurons exposed to NMDA, raising the possibility that NMDA receptor-mediated neurotoxicity contributes to selective neuronal death in HD [Koh, Peters, and Choi, *Science* 234:73-76 (1986); .Beal, Kowall, Ellison, Mazurek, Swartz, and Martin, *Nature* 321:168-171 (1986); Beal, Ferrante, Swartz, and Kowall: *J.Neurosci.* 11:1649-1659 (1991)].

### <Free radicals and Brain Diseases>

Free radicals are produced in degenerating brain areas following hypoxic-ischemia or traumatic brain and spinal cord injuries [Hall and Braughler, *Free Radic.Biol.Med.* 6:303-313 (1989); Anderson and Hall, *Ann.Emerg.Med.* 22:987-992 (1993); Siesjo and Siesjo, *Eur.J.Anaesthesiol.* 13:247-268(1996); Love, *Brain Pathol.* 9:119-131 (1999)]. Antioxidants or maneuvers scavenging free radicals attenuate brain damages by hypoxic-ischemia or traumatic injuries [Faden, *Pharmacol.Toxicol.* 78:12-17 (1996); Zeidman, Ling, Ducker, and Ellenbogen, *J.Spinal.Disord.* 9:367-380 (1996); Chan, *Stroke* 27:1124-1129 (1996); Hall, *Neurosurg.Clin.N.Am.* 8:195-206 (1997)]. Extensive evidence supports that free radicals can be produced in brain areas undergoing degeneration in neurodegenerative diseases possibly due to point mutations in Cu/Zn superoxide dismutase in ALS, decreased glutathione and increased iron in PD, accumulation of iron in AD, or mitochondrial dysfunction in HD [Rosen, Siddique, Patterson, Figlewicz, Sapp, Hentati, Donaldson, Goto, O'Regan, and Deng. *Nature* 362:59-62 (1993); Jenner and Olanow, *Neurology* 47:S161-S170 (1996); Smith, Harris, Sayre, and Perry, *Proc.Natl.Acad.Sci. U.S.A.* 94:9866-9868 (1997); Browne, Ferrante, and Beal, *Brain Pathol.* 9:147-163 (1999)]. Accordingly, antioxidants have been neuroprotective against such neurodegenerative diseases [Jenner, *Pathol. Biol. (Paris.)* 44:57-64 (1996); Beal, *Ann. Neurol.* 38:357-366 (1995); Prasad, Cole, and Kumar. *J.Am.Coll.Nutr.* 18:413-423 (1999); Eisen and Weber, *Drugs Aging* 14:173-196 (1999); Grundman, *Am.J. Clin.Nutr. 71: 630S.-636S (2000)].*

### <Zinc and Brain Diseases>

Zn²⁺ mediates neurodegenerative process observed in seizure, ischemia, trauma, and Alzheimers disease (AD). The central administration of kainate, a seizure-inducing excitotoxin, causes the translocation of Zn²⁺ into postsynaptic degenerating neurons in several forebrain areas [Frederickson, Hernandez, and McGinty. *Brain Res.* 480:317-321 (1989)]. Blockade of Zn²⁺ translocation with Ca-EDTA attenuates neuronal loss following a transient forebrain ischemia or traumatic brain injury [Koh, Sub, Gwag, He, Hsu and Choi, *Science* 272: 1013-1016 (1996); Suh, Chen, Motamedi, Bell, Listiak, Pons, Danscher, and Frederickson, *Brain Res.* 852 :268-273 *(2000)*]*.* Zn²⁺ is observed in the extracellular plaque and degenerating neurons in AD, which likely contributes to neuronal degeneration in AD [Bush, Pettingell, Multhaup, Paradis, Vonsattel, Gusella, Beyreuther, Masters, and Tanzi, *Science* 265:1464-1467 (1994); Suh, Jensen, Jensen, Silva, Kesslak, Danscher, and Frederickson. *Brain Res. 852:274-278* 852 *(2000)].*

The present invention relates to a compound represented by the following formula : 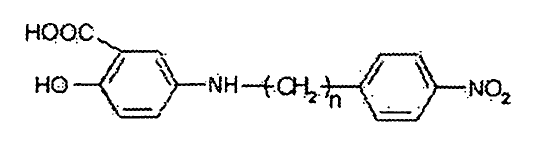 wherein, n is an integer of 2 or 3.

The present invention further relates to a pharmaceutical composition for protecting central neurons from acute or chronic injuries to central nervous system(CNS) comprising one or more of compounds represented by the following formula (I) or pharmaceutically acceptable salts thereof as an effective component: 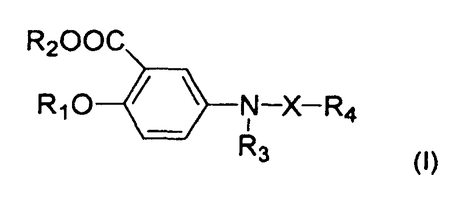 wherein,
X is CO, SO₂ or (CH₂)ₙ (where n is an integer of 1 to 5, inclusive);
R₁ is hydrogen, alkyl or alkanoyl;
R₂ is hydrogen or alkyl;
R₃ is hydrogen or an acetoxy group; and
R₄ is phenyl group which is unsubstituted or substituted with one or more of the group consisting of nitro, halogen, haloalkyl, and C₁- C₅ alkoxy; or a pharmaceutically-acceptable salt thereof.

It is preferred that said CNS injuries result from ischemia, hypoxia, hypoglycemia, traumatic brain injury, traumatic spinal cord injury, epilepsy, Huntington's disease, Parkinson's disease, Alzheimer's disease, or Amytrophic lateral sclerosis.

It is also preferred that said CNS injuries are caused by activation of N-methyl-D-aspartate (NMDA) glutamate receptors, entry and accumulation of Zn²⁺, or free radicals.

Furthermore, it is preferred that said composition attenuates NMDA neurotoxicity, Zn²⁺ neurotoxicity, and blocks free radical neurotoxicity as a direct antioxidant.

Finally, it is preferred that the compounds according to the above described composition are selected from the group consisting of
5-benzylaminosalicylic acid (BAS),
5-(4-nitrobenzyl)aminosalicylic acid (NBAS),
5-(4-chlorobenzyl)aminosalicylic acid (CBAS),
5-(4-trifluoromethylbenzyl)aminosalicylic acid (TBAS),
5-(4 fluorobenzyl)aminosalicylic acid (FBAS),
5-(4-methoxybenzyl)aminosalicylic acid (MBAS)
5-(4-pentafluorobenzyl)aminosalicylic acid (PBAS),
5-(4-nitrobenzyl)amino-2-hydroxy ethylbenzoate,
5-(4-nitrobenzyl)-*N*- acetylamino 2-hydroxy ethylbenzoate,
5-(4-nitrobenzyl)-*N*-acetylamino-2-acetoxy ethylbenzoate,
5-(4-nitrobenzoyl)aminosalicylic acid,
5-(4-nitrobenzenesulfonyl)aminosalicylic acid,
5-[2-(4-nitrophenyl)ethyl)aminosalicylic acid, and
5-[3-(4-nitrophenyl)-n-propyl)aminosalicylic acid, or a pharmaceutically-acceptable salt thereof.

### BRIEF DESCRIPTION OF THE DRAWINGS

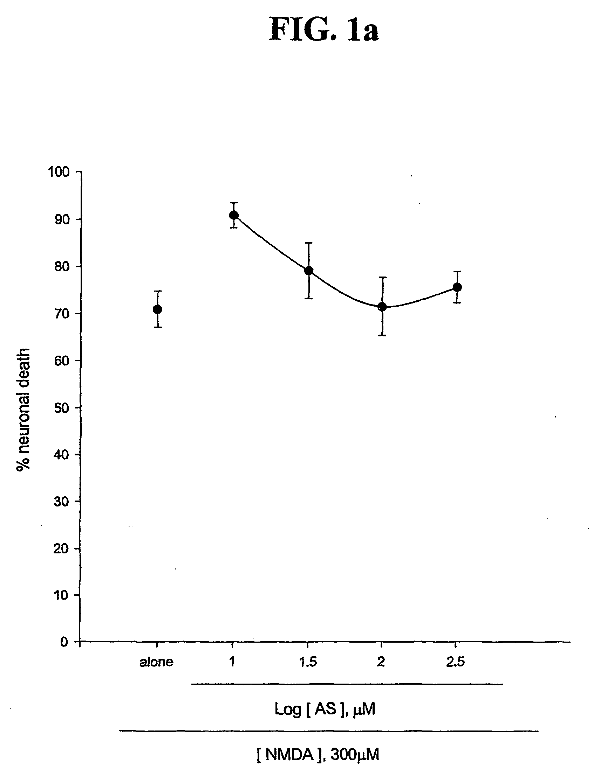

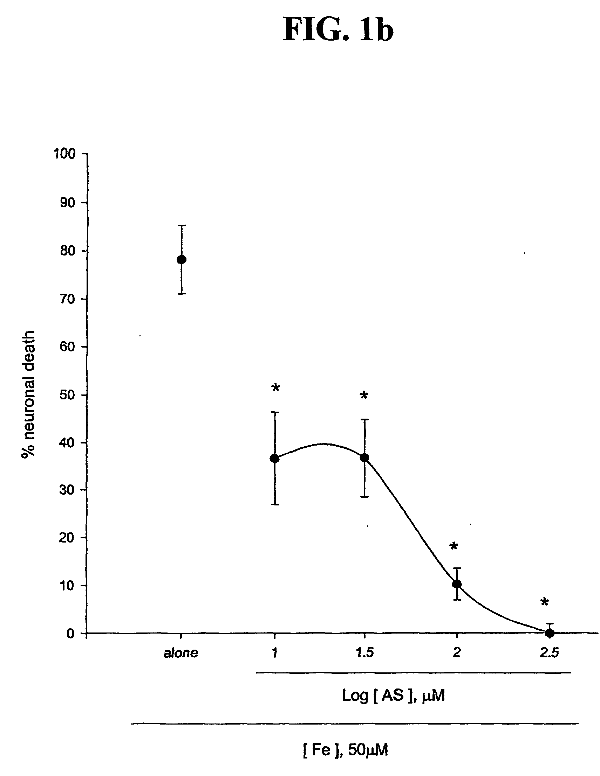

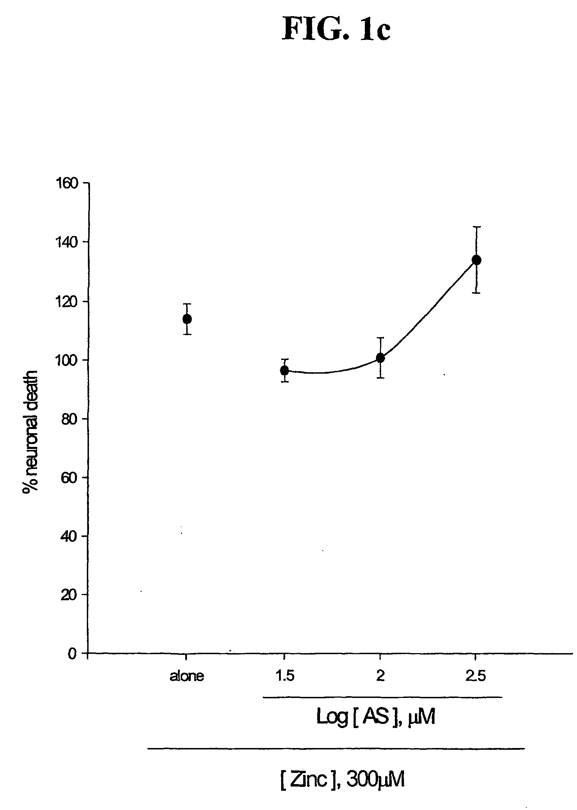

*Fig. 1.* Mouse cortical cell cultures (DIV 12 - 14) were exposed to 300 µM NMDA for 10 min (1a), continuously to 50 µM Fe²⁺ (1b), or 300 µM Zn²⁺ for 30 min (1c), alone or with inclusion of 10 - 300 µM 5-amino salicylic acid (AS). Neuronal death was analyzed 24 hr later by measuring levels of LDH released into the bathing medium, mean ± SEM (n = 9 - 12 (1a), n = 4 - 8 (1b) or n = 4 (1c) culture wells per condition), scaled to mean LDH efflux value 24 hr after sham wash (=0) and continuous exposure to 500 µM NMDA (=100). * indicates significant difference from relevant control at *p* < 0.05 using ANOVA and Student-Neuman-Keuls' test.

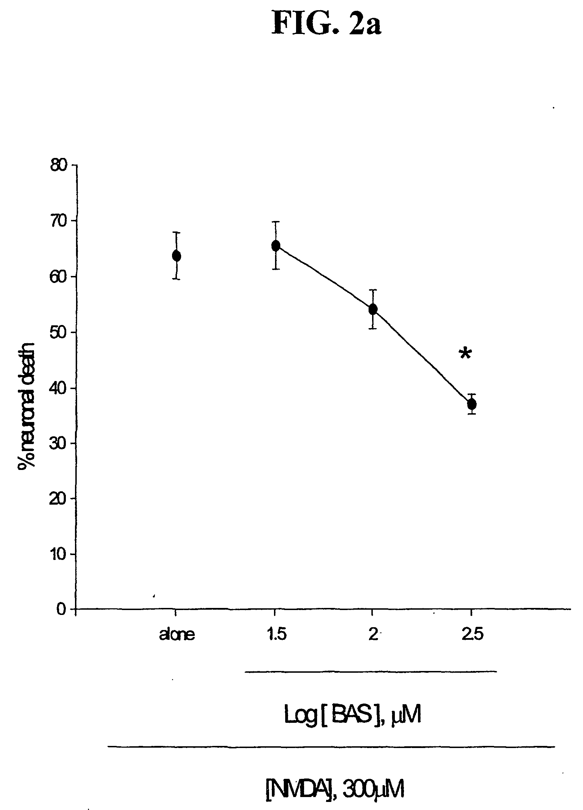

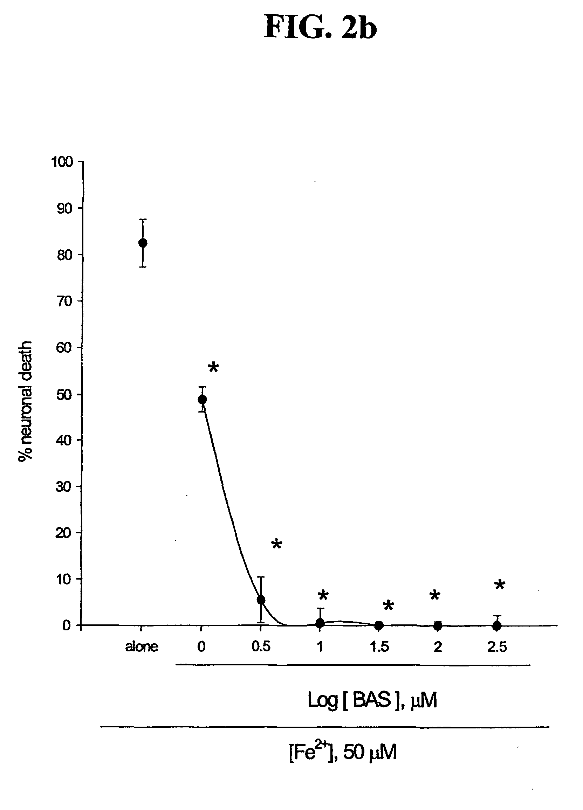

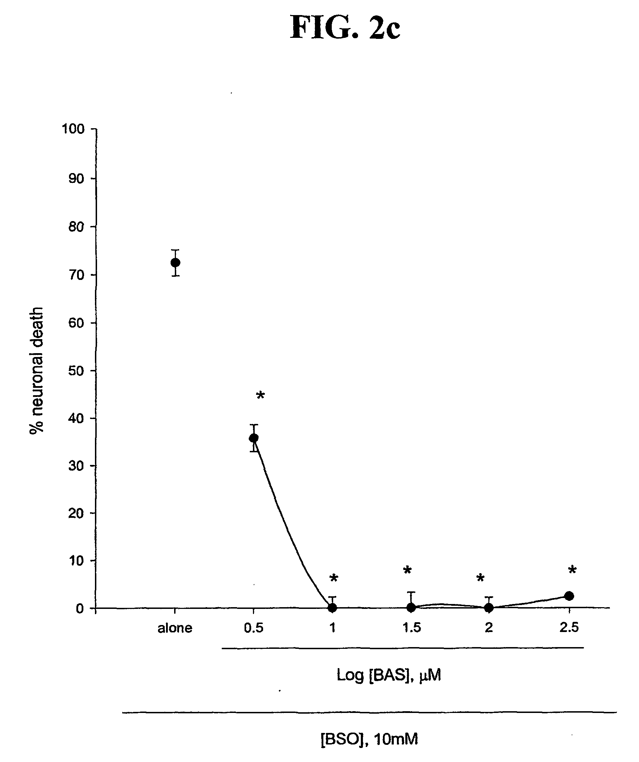

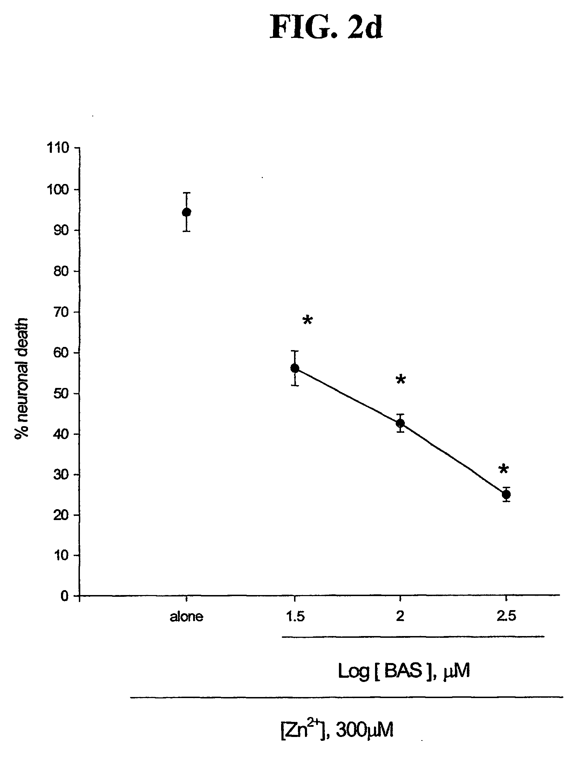

*Fig.* 2. Mouse cortical cell cultures (DIV 12 - 14) were exposed to 300 µM NMDA for 10 min (2a), continuously to 50 *µ*M Fe²⁺ (2b) or 10 mM BSO (2c), or 300 *µ*M Zn²⁺ for 30 min (2d), alone or with inclusion of indicated doses of 5-benzylamino salicylate (BAS). Neuronal death was analyzed 24 hr later by measuring levels of LDH released into the medium, mean ± SEM ( n = 7 - 8 (2a), n = 3 - 6 (2b), n = 4 (2c), or n = 4 (2d) culture wells per condition). * indicates significant difference from relevant control, at *p* < 0.05 using ANOVA and Student-Neuman-Keuls test.

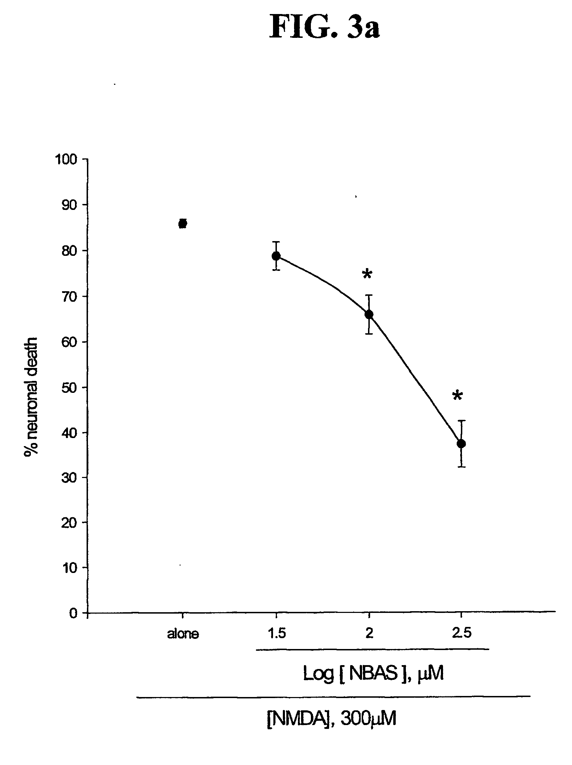

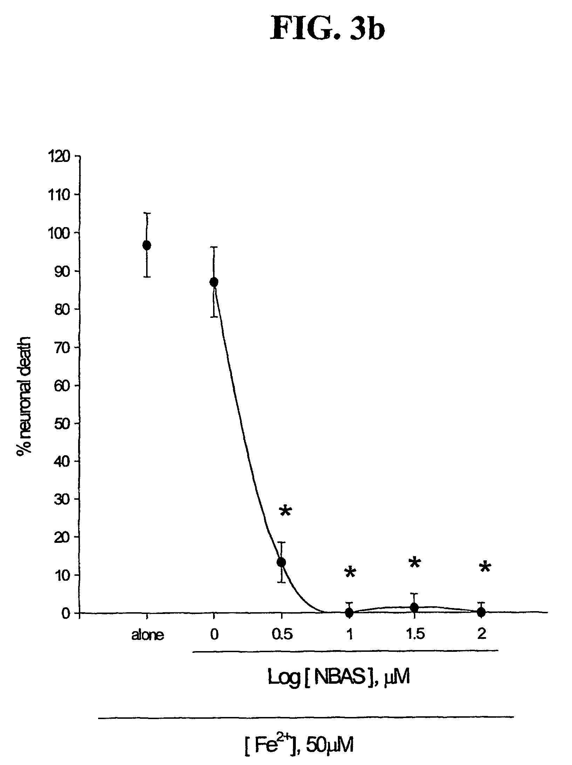

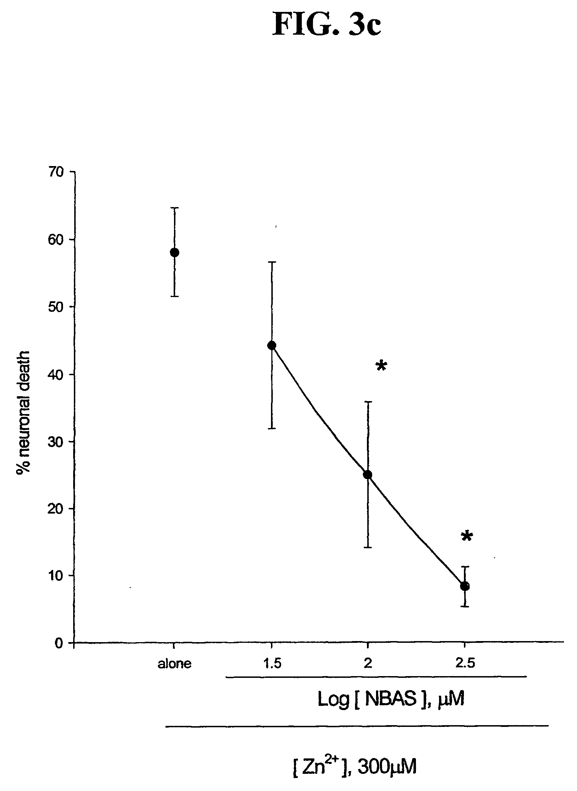

*Fig. 3*. Mouse cortical cell cultures (DIV 12 - 14) were exposed to 300 *µ*M NMDA for 10 min (3a), continuously to 50 *µ*M Fe²⁺ (3b), or 300 *µ*M Zn²⁺ for 30 min (3c), alone or with inclusion of indicated doses of 5-(4-nitrobenzyl)aminosalicylic acid (NBAS). Neuronal death was analyzed 24 hr later by measuring levels of LDH released into the medium, mean ± SEM ( n = 7 - 8 (3a), n = 3 - 6 (3b), or n = 4 (3c) culture wells per condition). * indicates significant difference from relevant control, at *p* < 0.05 using ANOVA and Student-Neuman-Keuls test.

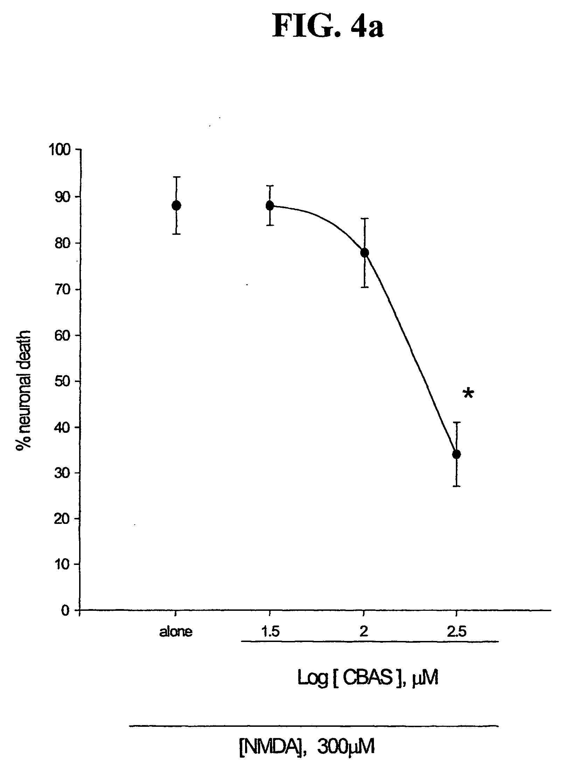

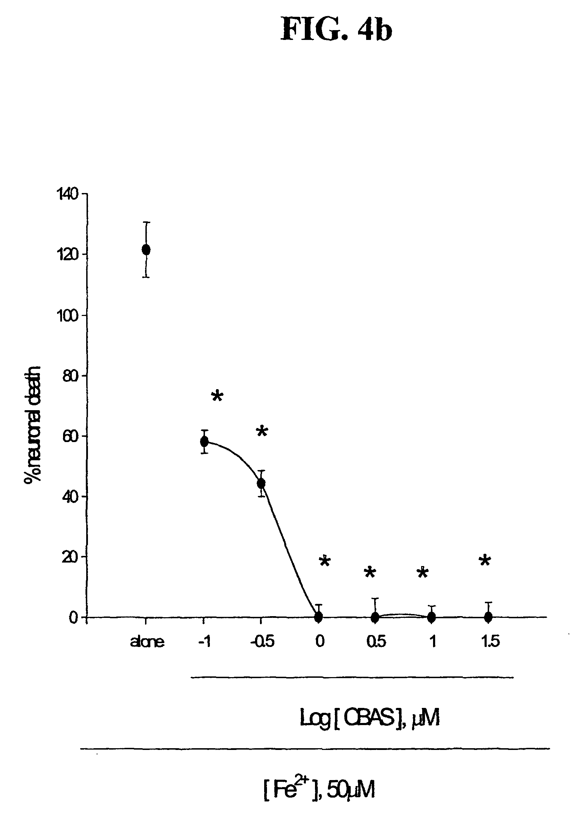

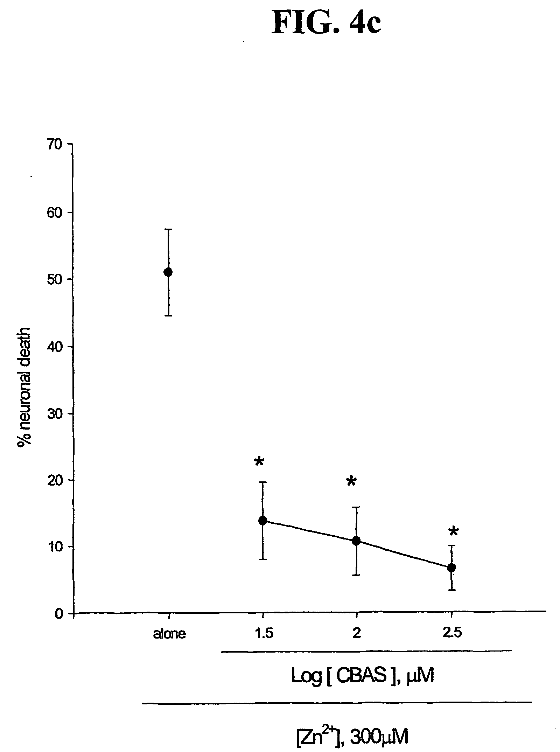

*Fig. 4.* Mouse cortical cell cultures (DIV 12 - 14) were exposed to 300 µM NMDA for 10 min (4a), continuously to 50 µM Fe²⁺ (4b), or 300 µM Zn²⁺ for 30 min (4c), alone or with inclusion of indicated doses of 5-(4-chlorobenzyl)aminosalicylic acid (CBAS). Neuronal death was analyzed 24 hr later by measuring levels of LDH released into the medium, mean ± SEM ( n = 3 - 4 (4a), n = 3 - 12 (4b), or n = 4 (4c) culture wells per condition). * indicates significant difference from relevant control, at *p* < 0.05 using ANOVA and Student-Neuman-Keuls test.

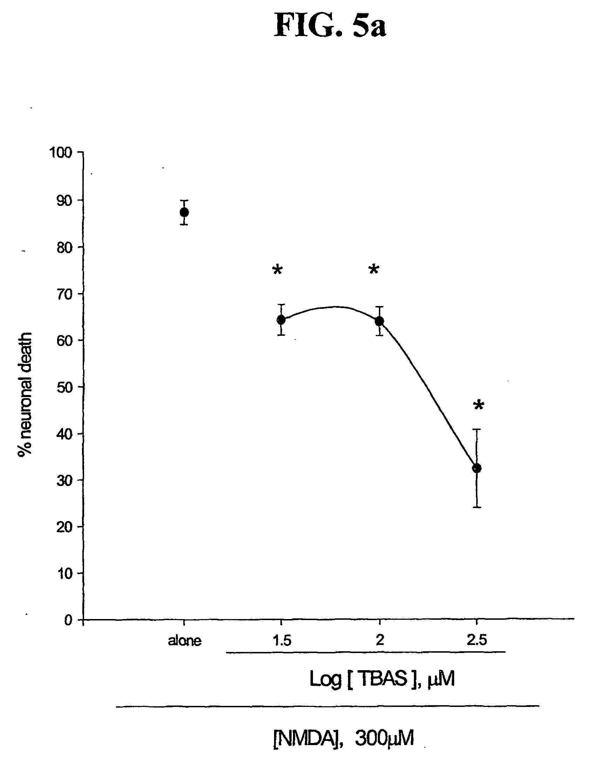

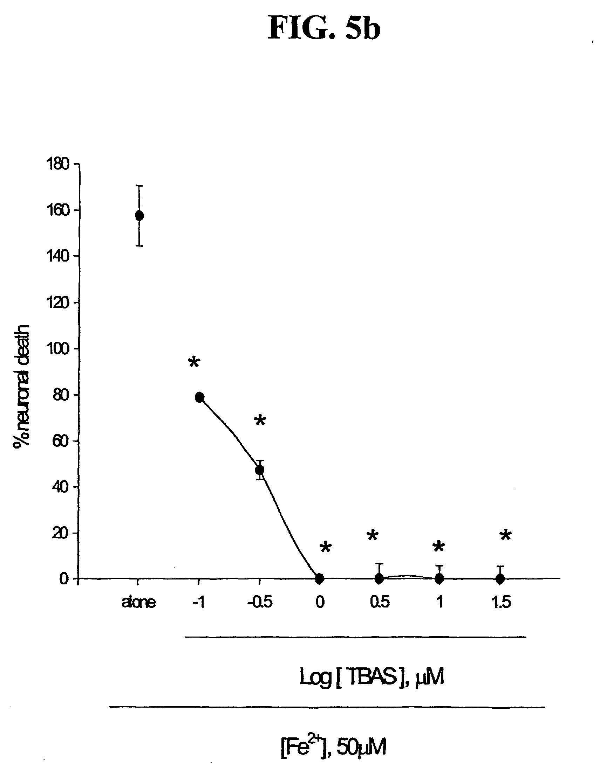

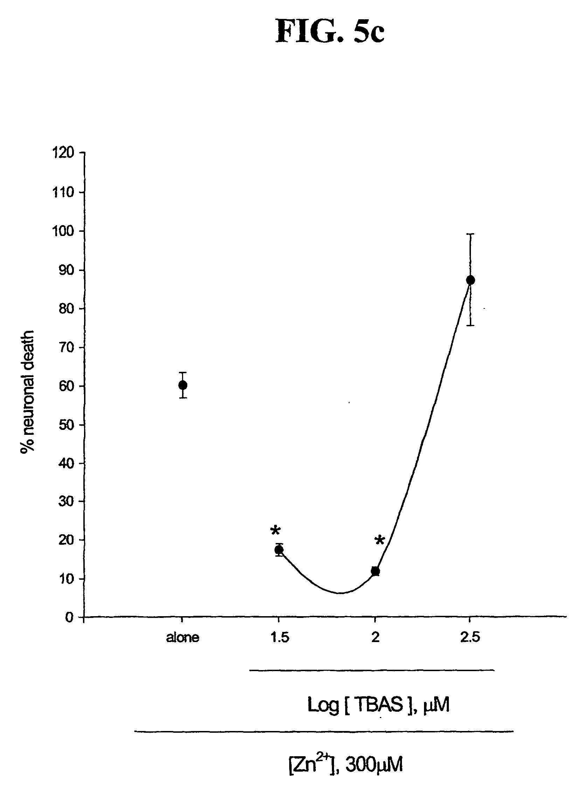

*Fig. 5*. Mouse cortical cell cultures (DIV 12 - 14) were exposed to 300 µM NMDA for 10 min (5a), continuously to 50 µM Fe²⁺ (5b), or 300 µM Zn²⁺ for 30 min (5c), alone or with inclusion of indicated doses of 5-(4-Trifluoromethylbenzyl)aminosalicylic acid (TBAS). Neuronal death was analyzed 24 hr later by measuring levels of LDH released into the medium, mean ± SEM ( n = 3 - 4 (5a), n = 3 - 11 (5b), or n = 4 (5c) culture wells per condition). * indicates significant difference from relevant control, at *p* < 0.05 using ANOVA and Student-Neuman-Keuls test.

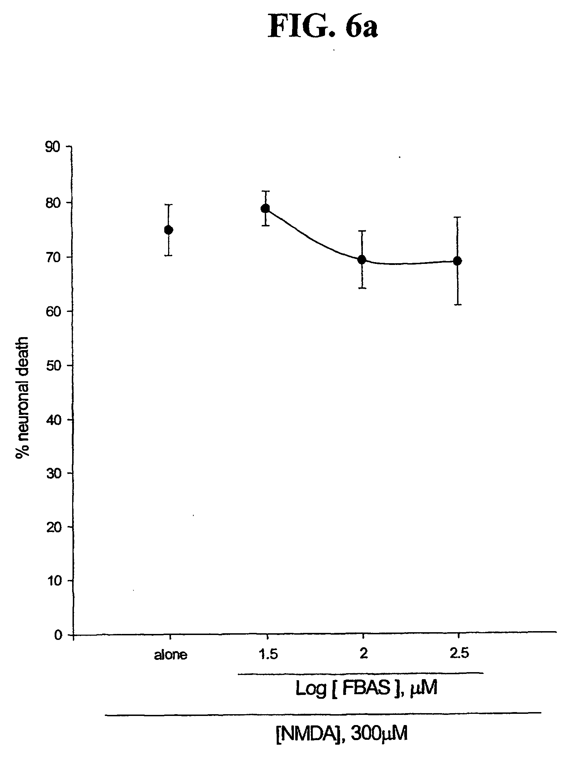

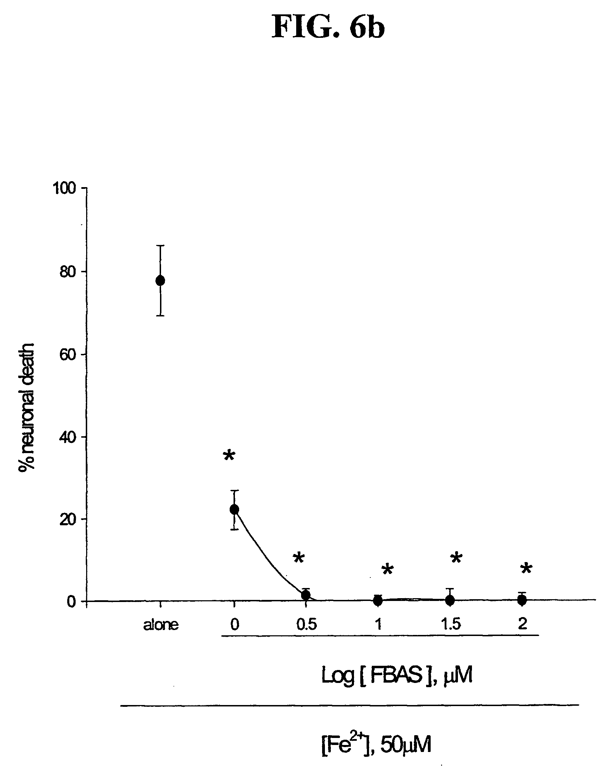

*Fig. 6*. Mouse cortical cell cultures (DIV 12 - 14) were exposed to 300 µM NMDA for 10 min (6a) or continuously to 50 µM Fe²⁺ (6b) alone or with inclusion of indicated doses of 5-(4-Fluorobenzyl)aminosalicylic acid(FBAS). Neuronal death was analyzed 24 hr later by measuring levels of LDH released into the medium, mean ± SEM ( n = 7 - 8 (6a) or n = 4 - 8 (6b) culture wells per condition). * indicates significant difference from relevant control, at *p* < 0.05 using ANOVA and Student-Neuman-Keuls test.

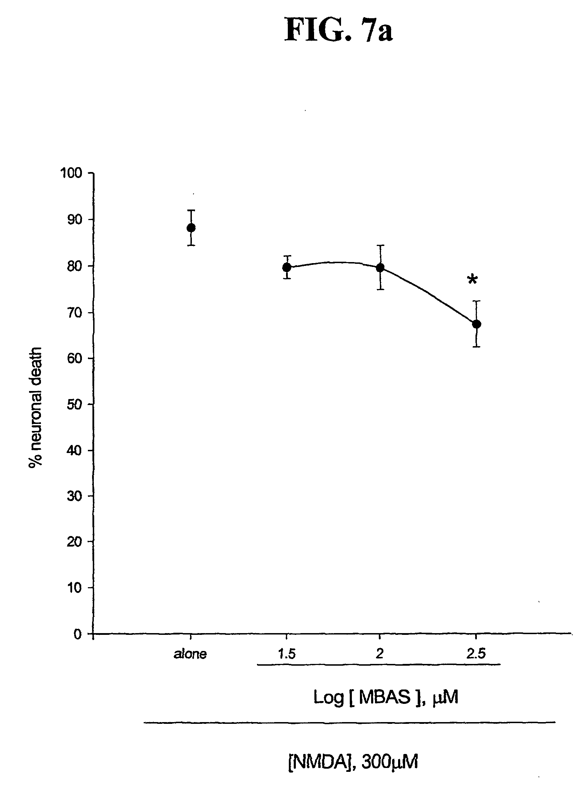

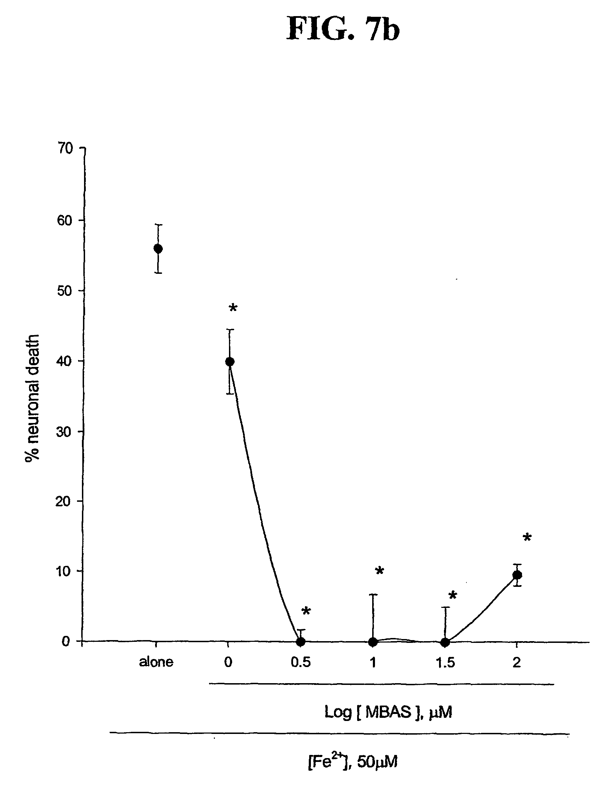

*Fig.* 7. Mouse cortical cell cultures (DIV 12 - 14) were exposed to 300 µM NMDA for 10 min (7a) or continuously to 50 µM Fe²⁺ (7b) alone or with inclusion of indicated doses of 5-(4-methoxybenzyl)aminosalicylic acid (MBAS). Neuronal death was analyzed 24 hr later by measuring levels of LDH released into the medium, mean ± SEM ( n = 7 - 8 (7a) or n = 4 - 8 (7b) culture wells per condition). * indicates significant difference from relevant control, at *p* < 0.05 using ANOVA and Student-Neuman-Keuls test.

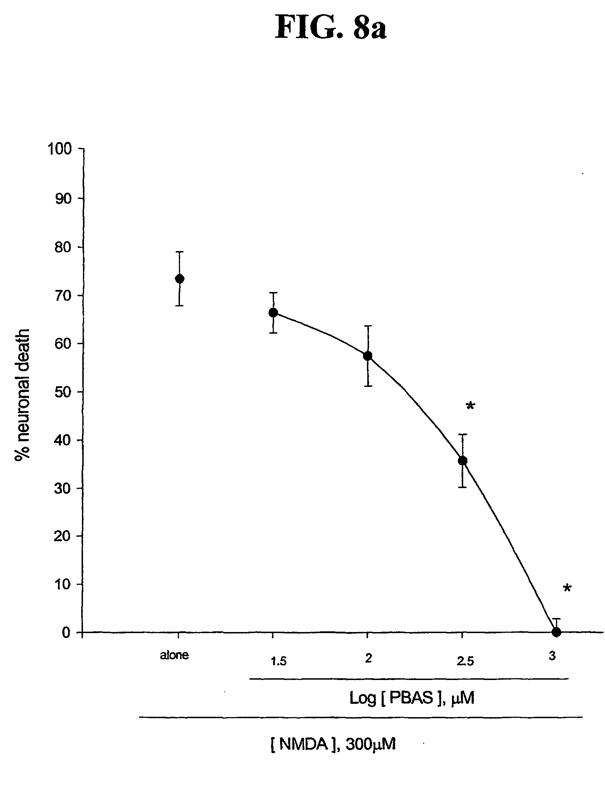

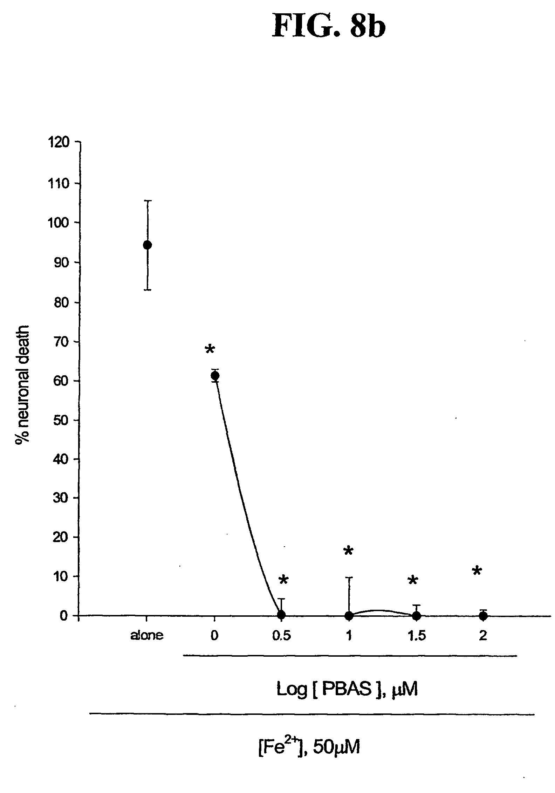

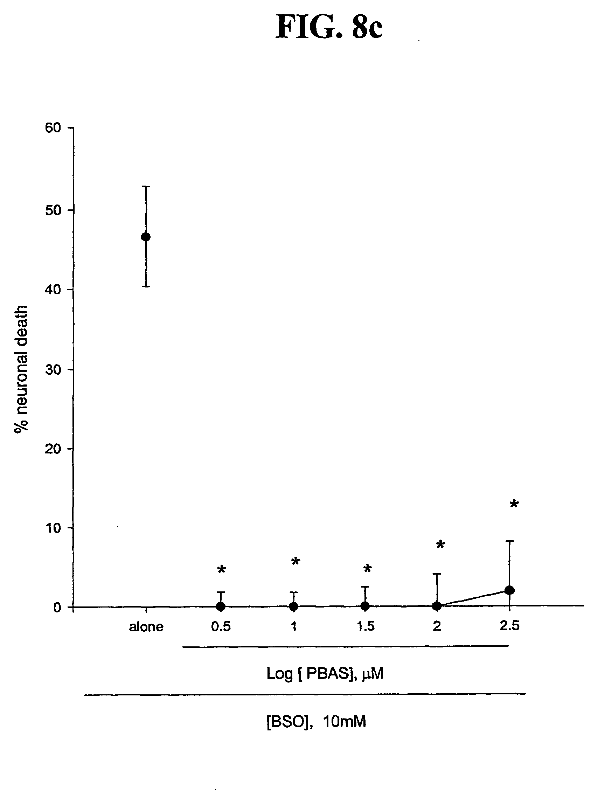

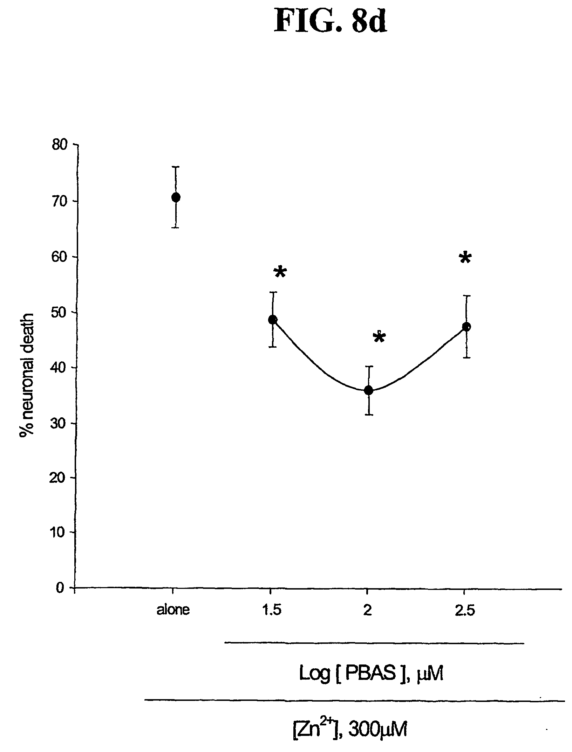

*Fig. 8*. Mouse cortical cell cultures (DIV 12 - 14) were exposed to 300 µM NMDA for 10 min (8a), continuously to 50 µM Fe²⁺ (8b) or 10 mM BSO (8c), or 300 µM Zn²⁺ for 30 min (8d), alone or with inclusion of indicated doses of 5-(pentafluorobenzyl)amino salicylic acid (PBAS). Neuronal death was analyzed 24 hr later by measuring levels of LDH released into the medium, mean ± SEM (n = 11 - 16 (8a), n = 3 - 6 (8b), n = 4 - 11 (8c), or n = 12 (8d) culture wells per condition). * indicates significant difference from relevant control, at *p* < 0.05 using ANOVA and Student-Neuman-Keuls test.

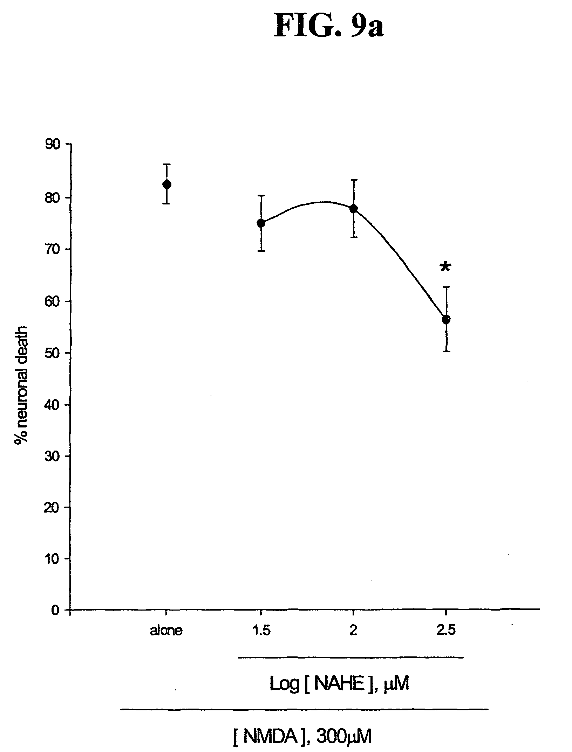

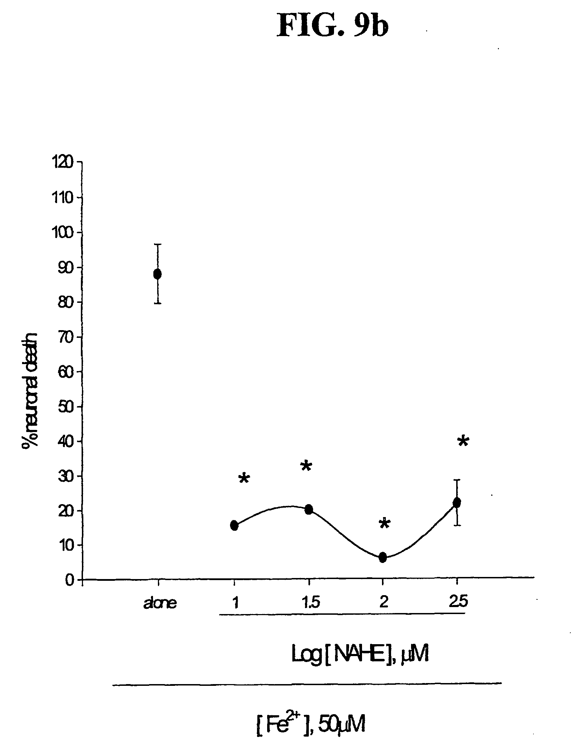

*Fig. 9.* Mouse cortical cell cultures (DIV 12 - 14) were exposed to 300 µM NMDA for 10 min (9a) or continuously to 50 µM Fe²⁺ (9b), alone or with inclusion of indicated doses of ethyl-5-(4-nitrobenzyl)amino-2-hydroxy ethylbenzoate (NAHE). Neuronal death was analyzed 24 hr later by measuring levels of LDH released into the medium, mean ± SEM (n = 10 - 12 (9a) or n = 3 - 4 (9b) culture wells per condition). * indicates significant difference from relevant control, at *p* < 0.05 using ANOVA and Student-Neuman-Keuls test.

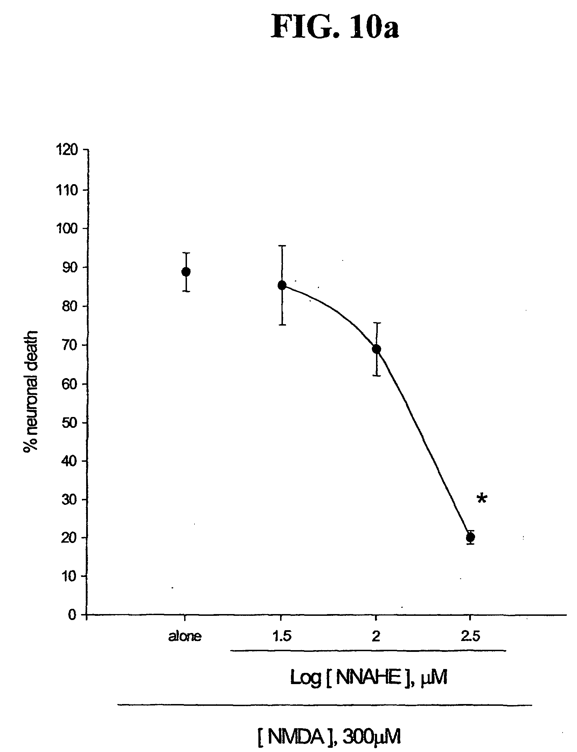

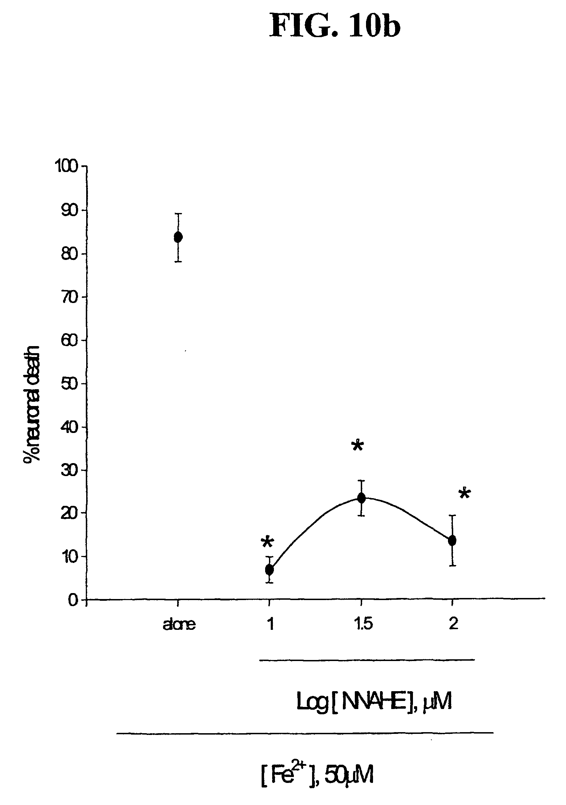

*Fig. 10.* Mouse cortical cell cultures (DIV 12 - 14) were exposed to 300 µM NMDA for 10 min (10a) or continuously to 50 µM Fe²⁺ (10b) alone or with inclusion of indicated doses of 5-(4-nitrobenzyl)-*N*-acetylamino-2-hydroxy ethylbenzoate (NNAHE). Neuronal death was analyzed 24 hr later by measuring levels of LDH released into the medium, mean ± SEM (n = 3 - 4 (10a) or n = 3 - 4 (10b) culture wells per condition). * indicates significant difference from relevant control, at *p* < 0.05 using ANOVA and Student-Neuman-Keuls test.

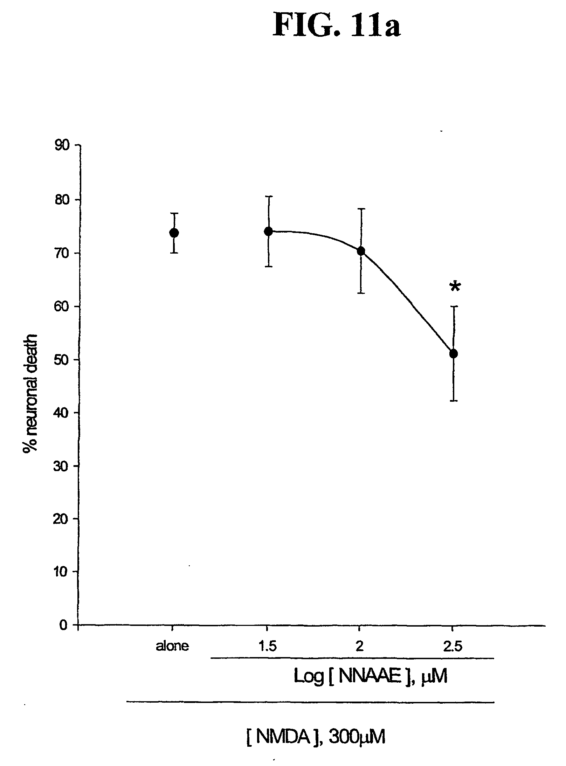

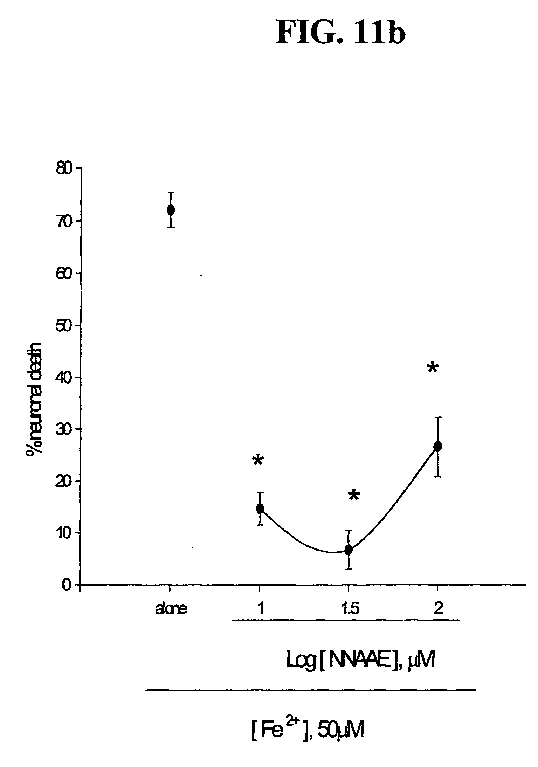

*Fig. 11.* Mouse cortical cell cultures (DIV 12 - 14) were exposed to 300 *µ*M NMDA for 10 min (11a) or continuously to 50 µM Fe²⁺ (11b), alone or with inclusion of indicated doses of 5-(4-nitrobenzyl)-*N*-acetylamino-2-acetoxy ethylbenzoate (NNAAE). Neuronal death was analyzed 24 hr later by measuring levels of LDH released into the medium, mean ± SEM (n = 10 - 12 (11a), n = 7 - 8 (11b), or n = 4 (11c) culture wells per condition). * indicates significant difference from relevant control, at p < 0.05 using ANOVA and Student-Neuman-Keuls test.

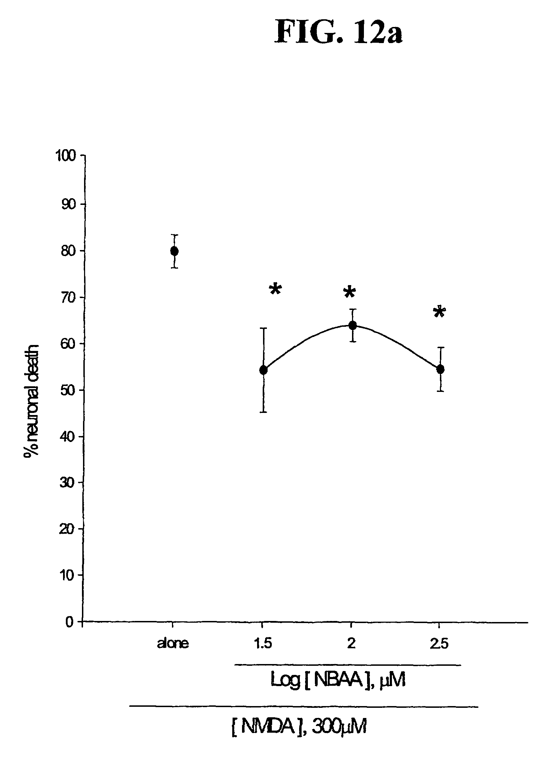

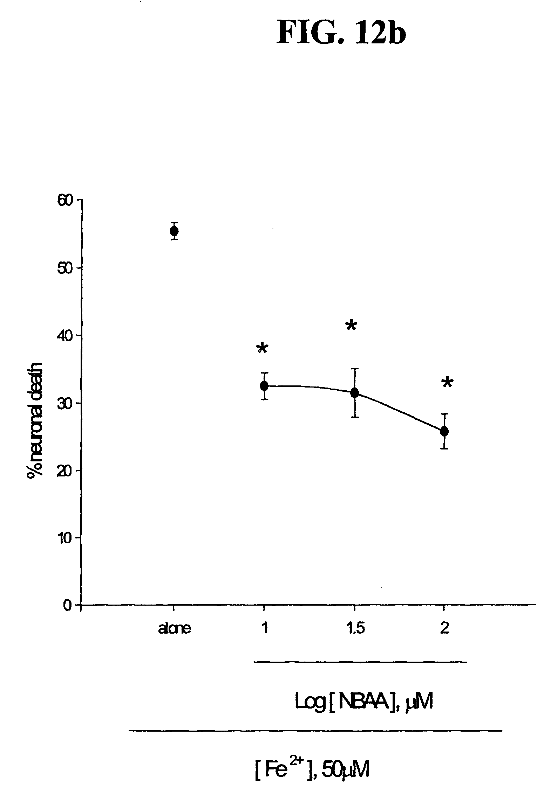

*Fig. 12.* Mouse cortical cell cultures (DIV 12 - 14) were exposed to 300 *µ*M NMDA for 10 min (12a) or continuously to 50 µM Fe²⁺ (12b), alone or with inclusion of indicated doses of 5-(4-nitrobenzonyl)aminosalicylic acid(NBAA). Neuronal death was analyzed 24 hr later by measuring levels of LDH released into the medium, mean ± SEM (n = 7 - 8 (12a) or n = 3 - 4 (12b) culture wells per condition). * indicates significant difference from relevant control, at *p* < 0.05 using ANOVA and Student-Neuman-Keuls test.

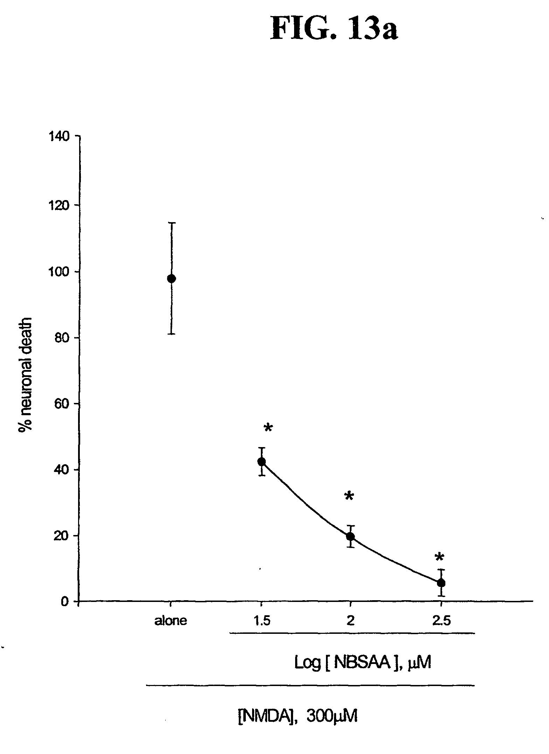

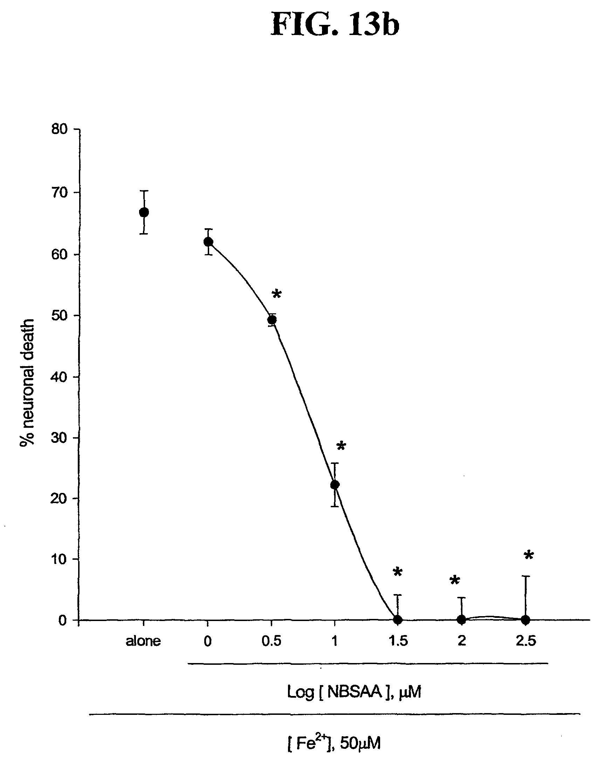

*Fig. 13*. Mouse cortical cell cultures (DIV 12 - 14) were exposed to 300 µM NMDA for 10 min (13a) or continuously to 50 µM Fe²⁺ (13b) alone or with inclusion of indicated doses of 5-(4-nitrobenzenesulfonyl)aminosalicylic acid(NBSAA). Neuronal death was analyzed 24 hr later by measuring levels of LDH released into the medium, mean ± SEM (n = 3 - 4 (13a) or n = 2 - 8 (13b) culture wells per condition). * indicates significant difference from relevant control, at *p* < 0.05 using ANOVA and Student-Neuman-Keuls test.

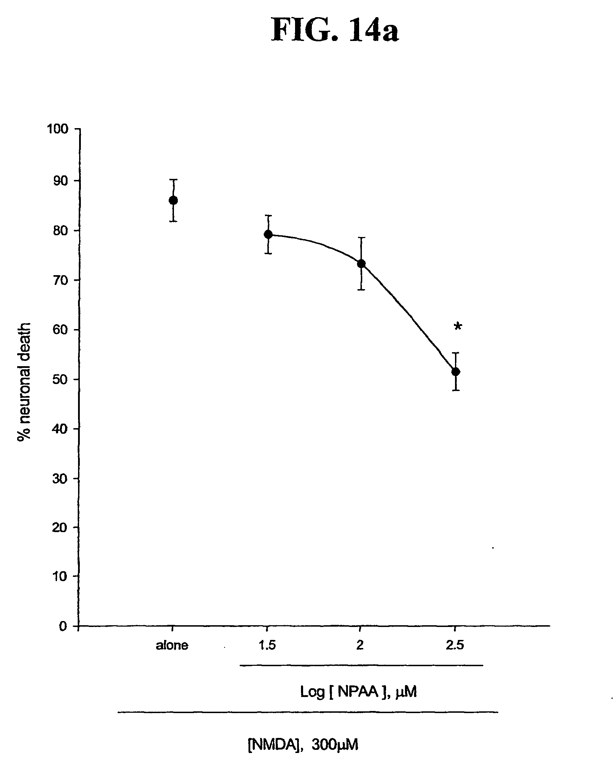

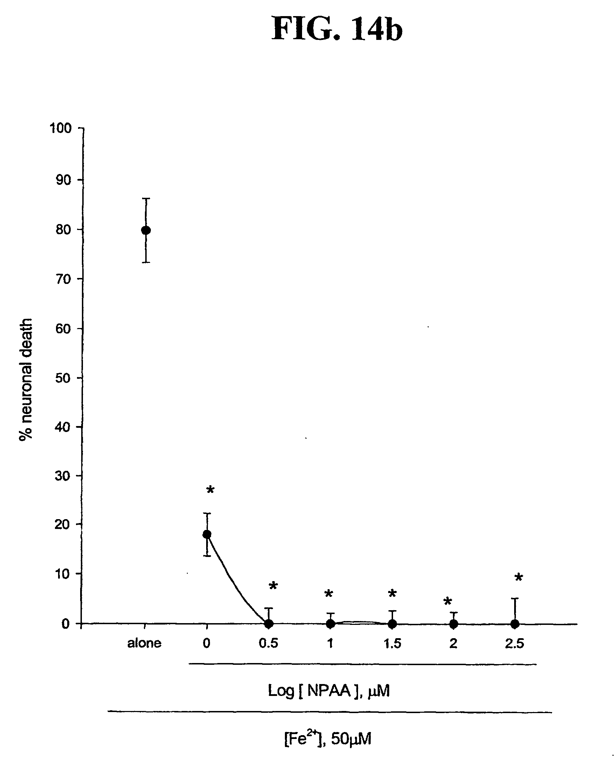

*Fig. 14*. Mouse cortical cell cultures (DIV 12 - 14) were exposed to 300 µM NMDA for 10 min (14a) or continuously to 50 µM Fe²⁺ (14b), alone or with inclusion of indicated doses of 5-[2-(4-nitrophenyl)-ethyl]aminosalicylic acid(NPAA). Neuronal death was analyzed 24 hr later by measuring levels of LDH released into the medium, mean ± SEM (n = 4 (14a) or n = 4 - 8 (14b) culture wells per condition). * indicates significant difference from relevant control, at *p* < 0.05 using ANOVA and Student-Neuman-Keuls test.

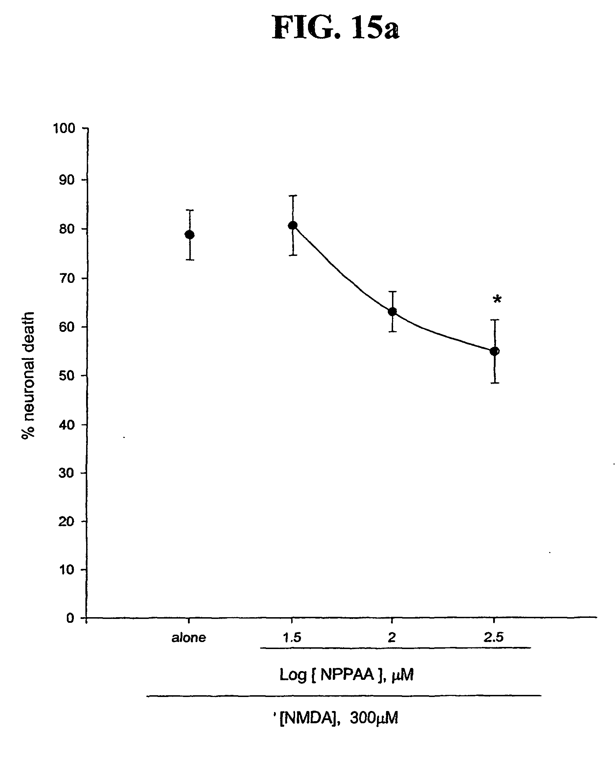

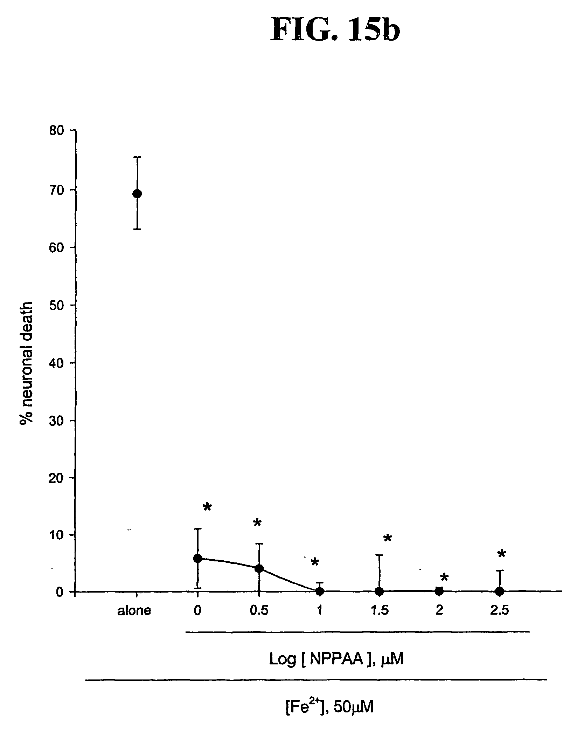

*Fig. 15.* Mouse cortical cell cultures (DIV 12 - 14) were exposed to 300 µM NMDA for 10 min (15a) or continuously to 50 µM Fe²⁺ (15b), alone or with inclusion of indicated doses of 5-[3-(4-nitrophenyl)-n-propyl]aminosalicylic acid (NPPAA). Neuronal death was analyzed 24 hr later by measuring levels of LDH released into the medium, mean ± SEM (n = 4 (15a) or n = 3 - 8 (15b) culture wells per condition). * indicates significant difference from relevant control, at *p* < 0.05 using ANOVA and Student-Neuman-Keuls test.

### DETAILED DESCRIPTION OF THE INVENTION

We have synthesized 5-benzylamino salicylic acid (BAS) and its derivatives and demonstrated that these synthetic compounds have multiple neuroprotective action. First, BAS and its derivatives attenuate NMDA neurotoxicity at doses of 100 - 1,000 uM. Second, BAS and its derivatives are antioxidants and block free radical neurotoxicity at doses of 1 - 300 uM. Finally, BAS and its derivatives attenuate Zn²⁺ neurotoxicity. These novel and multiple neuroprotective effects of BSA and its derivatives are merited to treat stroke, traumatic brain and spinal cord injury, epilepsy, and neurodegenerative diseases that are accompanied by excitotoxicity, Zn²⁺ neurotoxicity, and free radical neurotoxicity.

The BAS and its derivatives may be synthesized from 5-aminosalicylic acid by reacting it with an appropriate compound. The following reaction schemes illustrate the synthesis of BAS and its derivatives.

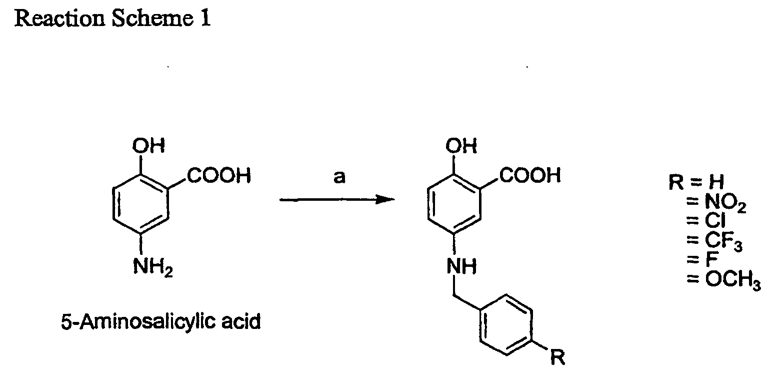

### Reagents (a) : Benzyl bromide, 4-nitrobenzyl bromide, 4-chlorobenzyl chloride, 4-(trifluoro methyl)benzyl chloride, 4-fluorobenzyl bromide, 4-methoxybenzyl chloride, N,N-dimethyl foramide (DMF), triethylamine, room temperature, 4 hr.

A preferred class of compounds within Formula (I) comprises those compounds wherein X is CO, SO₂ or (CH₂)ₙ (where n is an integer of 1 - 5, inclusive); R₁ is hydrogen, C₁- C₅ alkyl or C₂ - C₅ alkanoyl; R₂ is hydrogen or C₁- C₅ alkyl; R₃ is hydrogen or an acetoxy group; and R₄ is phenyl group which is unsubstituted or substituted with one or more selected from the group consisting of nitro, halogen, haloalkyl, and C₁ - C₅ alkoxy; or a pharmaceutically-acceptable salt thereof.

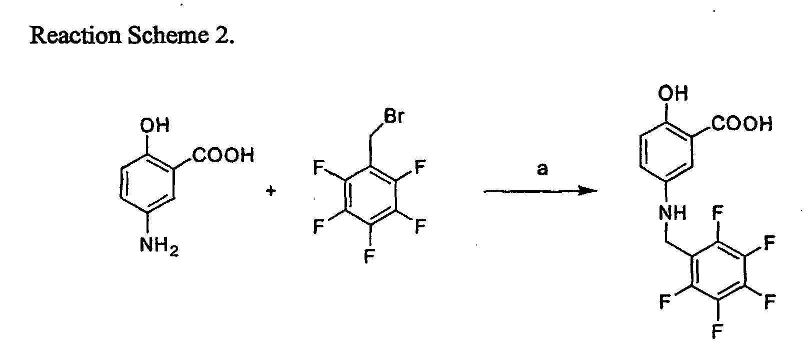

### Reagents (a) : N,N-dimethylforamide (DMF), triethylamine, room temperature, 4hr.

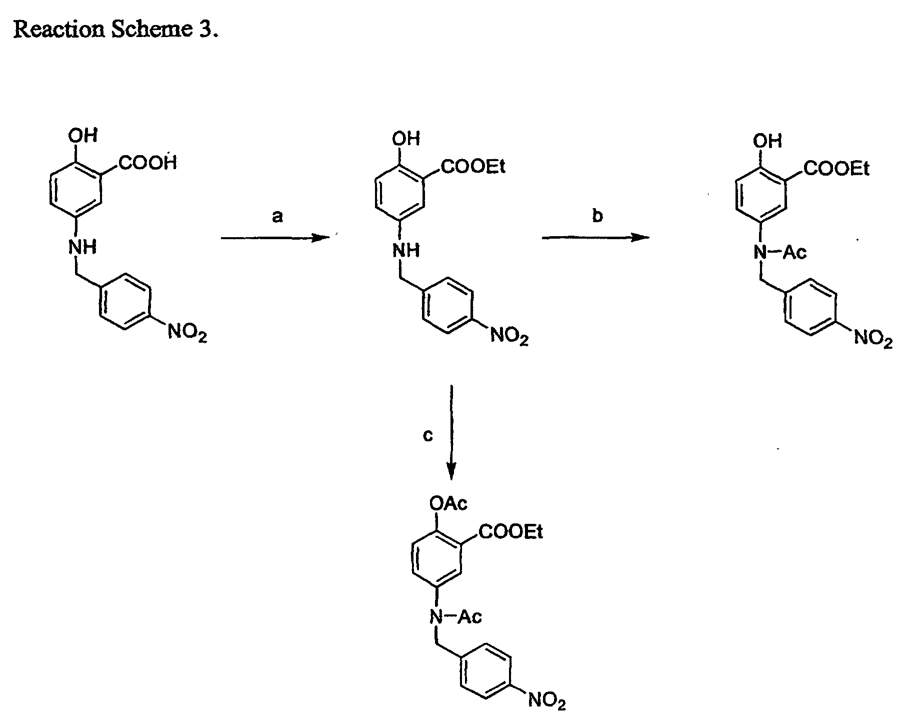

### Reagents (a) EtOH, H₂SO₄, reflux, 6hr.

(b) Acetic anhydride, MeOH, 0°C, 30 min.
(c) Acetic anhydride, H₂SO₄, 0°C, 30 min.

A more preferred class of compounds within Formula (I) encompasses those compounds wherein X is CO, SO₂ or (CH₂)ₙ (where n = 1,2,3); R₁ is hydrogen, C₁- C₃ alkyl or C₂ - C₃ alkanoyl; R₂ is hydrogen or C₁- C₃ alkyl; R₃ is hydrogen or an acetoxy group; R₄ is phenyl group which is unsubstituted or substituted with one or more selected from the group consisting of nitro, halogen, halo(C₁- C₃)alkyl and C₁- C₃ alkoxy; or a pharmaceutically- acceptable salt thereof.

### Example 5.

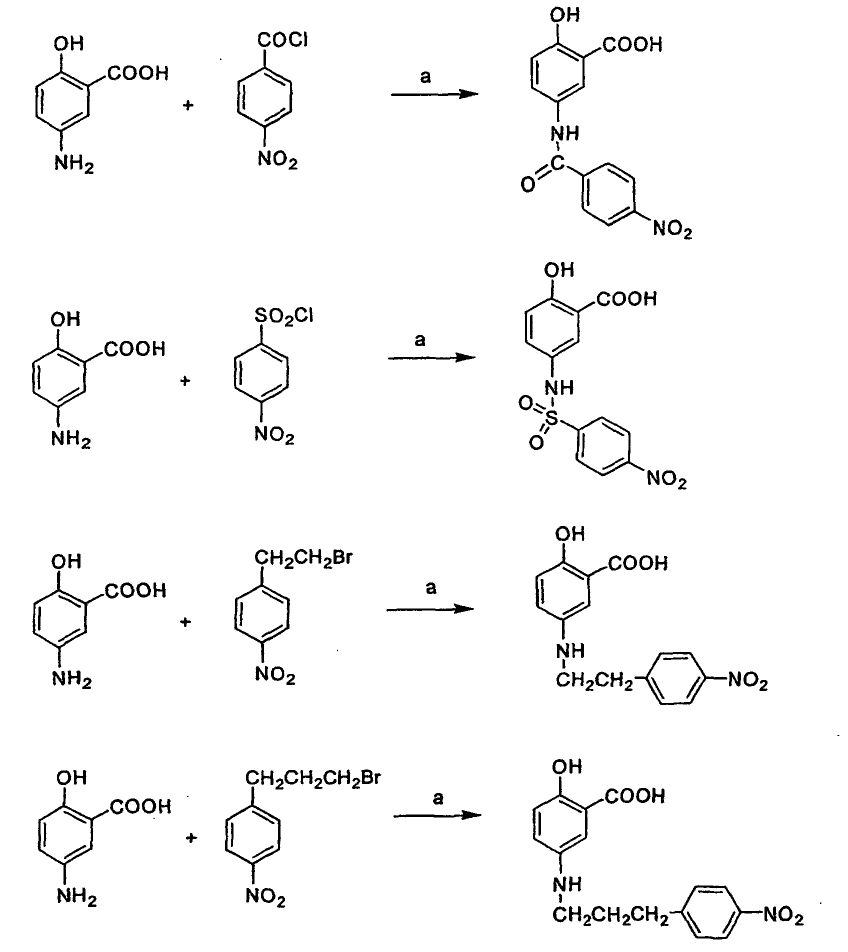

### Reagents (a) N,N-dimethylforamide (DMF), triethylamine, room temperature, 4hr.

Specific compounds of interest within Formula 5) are as follows:
5-benzylaminosalicyclic acid (BAS),
5-(4-nitrobenzyl)aminosalicyclic acid (NBAS),
(5-(4-chlorobenzyl)aminosalicyclic acid (CBAS),
(5-(4-trifluoromethylbenzyl)aminosalicyclic acid (TBAS),
(5-(4-fluorobenzyl)aminosalicyclic acid (FBAS),
5-(4-methoxybenzyl)aminosalicylic acid (MBAS)
5-(pentafluorobenzyl)aminosalicylic acid (PBAS),
5-(4-nitrobenzyl)amino-2-hydroxy ethylbenzoate(NAHE),
5-(4-nitrobenzyl)-N-acetylamino-2-hydroxy ethylbenzoate(NNAHE),
5-(4-nitrobenzyl)-N acetylamino-2-acetoxy ethylbenzoate(NNAAE),
5-(4-mtrobenzoyl)ammosalicylic acid(NBAA),
5-(4-nitrobenzenesulfonyl)aminosalicyhc acid(NBSAA),
5-[2-(4-nitrophenyl)-ethyl]aminosalicylic acid(NPAA), and
5-[3-(4-nitrophenyl)-n propyl]aminosalicylic acid(NPPAA), or a pharmaceutically-acceptable salt thereof.

The term "pharmaceutically-acceptable salts" embraces salts commonly used to form alkali metal salts and to form addition salts of free acids or free bases. The nature of the salt is not critical, provided that it is pharmaceutically acceptable, and acids or bases which may be employed to form such salts are, of course, well known to those skilled in the art Examples of acids which may be employed to form pharmaceutically acceptable acid addition salts include such inorganic acids as hydrochloric acid, sulfuric acid and phosphoric acid and such organic acids as maleic acid, succinic acid and citric acid. Other salts include salts with alkali metals or alkaline earth metals, such as sodium, potassium, calcium and magnesium, or with organic bases, such as dicyclohexylamine. All of these salts may be prepared by conventional means from the corresponding compound of Formula (I) by reacting, for example, the appropriate acid or base with the compound of Formula (I).

The Synthesis Examples show the exemplary method for the preparation of the representative compounds (I).

### Synthesis Example 1: Preparation of 5-benzylaminosalicylic acid (BAS)

To a solution of 5-aminosalicylic acid (2.0 g, 13 mmole, purchased from Aldrich Chemical Company, USA) and triethylamine in dried DMF (25 ml) was added benzyl bromide (2.68 g, 1.90 ml, 15.6 mmole) at room temperature under a nitrogen atmosphere. The reaction mixture was stirred for 4 hr at room temperature. Ice chip was added to the reaction mixture and then solvent was removed in *vacuo.* The reaction mixture was diluted with water and then extracted with ethyl acetate. The organic layer was washed with water and brine, and then dried over anhydrous. MgSO₄. After evaporation of the solvent, the residue was purified by column chromatography and recrystallized from methanol/ethyl acetate/hexane (1:3:1) to give 3.6 g (73% yield) of 5-benzylaminosalylic acid as a white solid. : mp 173.5-174.5 °C (decompose).

| Elemental analysis for C₁₄H₁₃NO₃. | | | |
|---|---|---|---|
| | %C | %H | %N |
| Calculated | 69.12 | 5.39 | 5.76 |
| Found | 69.30 | 5.18 | 5.63 |

### Synthesis Example 2 : Preparation of 5-(4-nitrobenzyl)aminosalicylic acid (NBAS)

By following the similar procedure in Synthesis Example 1 by using 5-aminosalicylic acid (2.00 g, 13.0 mmole) and 4-nitrobenzyl bromide (3.38 g, 15.6 mmole), 2.90 g (79% yield) of 5-(4-nitrobenzyl)aminosalicylic acid was obtained as a pale yellow solid. : mp 211-212 °C.

| Elemental analysis for C₁₄H₁₃N₂O₅. | | | |
|---|---|---|---|
| | % C | % H | % N |
| Calculated | 58.33 | 4.20 | 9.72 |
| Found | 58.38 | 4.21 | 9.71 |

### Synthesis Example 3 : Preparation of 5-(4-chlorobenzyl)aminosalicylic acid (CBAS)

By following the similar procedure in Synthesis Example 1 by using 5-aminosalicylic acid (500 mg, 3.26 mmole) and 4-chlorobenzyl chloride (630 mg, 3.91 mmole), 480 mg (53% yield) of 5-(4-chlorobenzyl)aminosalicylic acid was obtained as a white solid. : mp 227-228 °C.

| Elemental analysis for C₁₄H₁₂CINO₃. | | | |
|---|---|---|---|
| | % C | %H | %N |
| Calculated | 60.55 | 4.36 | 5.04 |
| Found | 60.43 | 4.21 | 5.02 |

Synthesis Example 4 : Preparation of 5-(4-trifluoromethylbenzyl)aminosalicylic acid (TBAS)

By following the similar procedure in Synthesis Example 1 by using 5-aminosalicylic acid (500 mg, 3.26 mmole) and 4-(trifluoromethyl)benzyl chloride (760 mg, 3.92 mmole), 510 mg (50% yield) of 5-(4-(trifluoromethyl)benzyl)aminosalicylic acid was obtained as a white solid. : mp > 188 °C (decompose).

| Elemental analysis for C₁₄H₁₂F₃NO₃. | | | |
|---|---|---|---|
| | %C | %H | %N |
| Calculated | 57.88 | 3.89 | 4.50 |
| Found | 57.61 | 3.98 | 4.44 |

### Synthesis Example 5 : Preparation of 5-(4-fluorobenzyl)aminosalicylic acid (FBAS)

By following the similar procedure in Synthesis Example 1 by using 5-aminosalicylic acid (500 mg, 3.26 mmole) and 4-fluorobenzyl bromide (740 mg, 3.92 mmole), 480 mg (44% yield) of 5-(4-fluorobenzyl)aminosalicylic acid was obtained as a white solid. : mp > 210 °C (decompose).

| Elemental analysis for C₁₄H₁₂FNO₃. | | | |
|---|---|---|---|
| | %C | %H | %N |
| Calculated | 64.36 | 4.63 | 5.36 |
| Found | 64.10 | 4.42 | 5.07 |

### Synthesis Example 6 : Preparation of 5-(4-methoxybenzyl)aminosalicylic acid (MBAS)

By following the similar procedure in Synthesis Example 1 by using 5-aminosalicylic acid (1.00 g, 6.53 mmole) and 4-methoxybenzyl chloride (1.23 g, 7.84 mmole), 890 mg (50% yield) of 5-(4-methoxybenzyl)aminosalicylic acid was obtained as a white solid. : mp 205-206 °C.

| Elemental analysis for C₁₅H₁₅NO₄. | | | |
|---|---|---|---|
| | % C | % H | % N |
| Calculated | 65.92 | 5.53 | 5.13 |
| Found | 65.83 | 5.45 | 5.07 |

### Synthesis Example 7: Preparation of 5-(pentafluombenzyl)aminosalicylic acid

By following the similar procedure in Synthesis Example 1 by using 5-aminosalicylic acid (500 mg, 3.26 mmole) and pentafluorobenzyl bromide (1.02 g, 3.92 mmole), 650 mg (60% yield) of 5-(pentafluorobenzyl)aminosalicylic acid was obtained as a white solid. : mp >190 °C (decompose).

| Elemental analysis for C₁₄H₈F₅NO₃. | | | |
|---|---|---|---|
| | %C | %H | %N |
| Calculated | 50.46 | 2.42 | 4.20 |
| Found | 50.53 | 2.19 | 4.23 |

Synthesis Example 8: Preparation of 5-(4-nitrobenzyl)amino-2-hydroxy ethylbenzoate

To a solution of 5-(4-nitrobenzyl)aminosalicylic acid (1.0 g, 3.4 mmole) in ethanol (35 ml) was carefully added Conc.H₂SO₄ (3.5 ml) at 0°C. The reaction mixture was stirred for 6 hr at 80 °C and cooled to room temperature. After the solvent was removed in *vacuo,* the reaction mixture was extracted with ethyl acetate. The organic layer was washed with H₂O, 10% NaHCO₃ solution, 5% HCl solution and brine. After the organic layer was dried over anhydrous MgSO₄, it was concentrated in *vacuo.* The residue was purified by column chromatography and recrystalized from ethyl acetate/hexane (1:2) to give 500 mg (46% yield) of 5-(4-nitrobenzyl)amino-2-hydroxy ethylbenzoate as a yellow solid : mp 106.5-107.5 °C.

| Elemental analysis for C₁₆H₁₆N₂O₅. | | | |
|---|---|---|---|
| | %C | %H | %N |
| Calculated | 60.75 | 5.10 | 8.86 |
| Found | 60.68 | 5.24 | 9.04 |

### Synthesis Example 9 :Preparation of 5-(4-nitrobenzyl)-N acetylamino-2 hydroxy ethylbenzoate

To a solution of 5-(4-nitrobenzyl)amino-2 hydroxy ethylbenzoate (500 mg, 1.58 mmole) in dried methanol (50 ml) was carefully added acetic anhydride (5 ml) at 0 °C under a nitrogen atmosphere. The reaction mixture was stirred for 2 hr at 10 °C. After ice chip was slowly added to the reaction mixture, the solvent was removed in *vacuo.* The reaction mixture was extracted with ethyl acetate and H₂O, and the organic layer was washed with H₂O, 10% NaHCO₃ (30 ml x 3) solution, 5% HCl (30 ml x 2) solution and brine. The organic solution was dried over anhydrous MgSO₄ and evaporated. The residue was purified by column chromatography and recrystalized from ethyl acetate/hexane (1:3) to give 400 mg (70 % yield) of 5-(4-nitrobenzyl)-*N*-acetylamino-2-hydroxy ethylbenzoate as a pale yellow solid. : mp 105.5-106.0 °C.

| Elemental analysis for C₁₈H₁₈N₂O₆. | | | |
|---|---|---|---|
| | %C | %H | %N |
| Calculated | 60.33 | 5.06 | 7.82 |
| Found | 60.54 | 5.35 | 8.12 |

### Synthesis Example 10 : Preparation of 5-(4-Nitrobenzyl)-N-acetylamino-2-acetoxy ethylbenzoate

To a solution of 5-(4-nitrobenzyl)amino-2-hydroxy ethylbenzoate (500 mg, 1.58 mmole) in acetic anhydride (10 ml) was carefully added conc.H₂SO₄ (0.5 ml) at 0 °C under a nitrogen atmosphere. The reaction mixture was stirred for 30 min at 10 °C. After ice chip was slowly added to the reaction mixture, the solvent was removed in *vacuo.* The reaction mixture was diluted with water and extracted with ethyl acetate. The organic layer was washed H₂O, 10% NaHCO₃ (30ml x 3) solution, 5% HCl (30ml x 2) solution and brine and then dried over anhydrous MgSO₄. After evaporation of the solvent, the residue was purified by column chromatography to give 450 mg (71% yield) of 5-(4-nitrobenzyl)-*N*-acetylamino-2-acetoxy ethylbenzoate as pale yellow oil.

| Elemental analysis for C₂₀H₂₀N₂O₇. | | | |
|---|---|---|---|
| | % C | % H | % N |
| Calculated | 60.00 | 5.03 | 7.00 |
| Found | 59.96 | 4.84 | 7.15 |

### Synthesis Example 11: Preparation of 5-(4-nitrobenzoyl)aminosalicylic acid

By following the similar procedure in Synthesis Example 1 by using 5-aminosalicylic acid (500 mg, 3.26 mmole) and 4-nitrobenzoyl chloride (700 mg, 3.77 mmole), 550 mg (56% yield) of 5-(4-nitrobenzoyl)aminosalicylic acid was obtained as a pale yellow solid. : mp 270-271 °C.

| Elemental analysis for C₁₄H₁₀N₂O₆. | | | |
|---|---|---|---|
| | %C | %H | %N |
| Calculated | 55.63 | 3.33 | 9.27 |
| Found | 55.82 | 3.43 | 9.08 |

### Synthesis Example 12 : Preparation of 5-(4-nitrobenzenesulfonyl)aminosalicylic acid

By following the similar procedure in Synthesis Example 1 by using 5-aminosalicylic acid (500 mg, 3.26 mmole) and 4-nitrobenzenesulsonyl chloride (720 mg, 3.26 mmole), 390 mg (35% yield) of 5-(4-nitrobenzenesulfonyl)aminosalicylic acid was obtained as a yellow solid. : mp 239-240 °C.

| Elemental analysis for C₁₃H₁₀N₂O₇S· | | | | |
|---|---|---|---|---|
| | %C | %H | %N | %S |
| Calculated | 46.15 | 2.98 | 8.28 | 9.48 |
| Found | 46.27 | 2.92 | 8.34 | 9.51 |

### Synthesis Example 13 : Preparation of 5-[2-(4-nitrophenyl)ethyl]aminosalicylic acid

By following the similar procedure in Synthesis Example 1 by using 5-aminosalicylic acid (500 mg, 3.26 mmole) and 4-nitrophenethyl bromide (900 mg, 3.92 mmole), 890 mg (50% yield) of 5-(4-nitrophenethyl)aminosalicylic acid was obtained as a pale yellow solid. : mp 234-236 °C.

| Elemental analysis for C₁₅H₁₄N₂O₅. | | | |
|---|---|---|---|
| | %C | %H | %N |
| Calculated | 59.60 | 4.67 | 9.27 |
| Found | 59.77 | 4.79 | 9.24 |

### Synthesis Example 14 : Preparation of 5-[3-(4-nitrophenyl)-n-propyl]aminosalicylic acid

By following the similar procedure in Synthesis Exmaple 1 by using 5-aminosalicylic acid (500 mg, 3.26 mmole) and 3-(4-nitrophenyl)propyl bromide (950 mg, 3.92 mmole), 520 mg (50% yield) of 5-[3-(4-nitrophenyl)-n-propyl]aminosalicylic acid was obtained as a pale yellow solid. : mp 229-231 °C.

| Elemental analysis for C₁₆H₁₆N₂O₅. | | | |
|---|---|---|---|
| | %C | %H | %N |
| Calculated | 60.75 | 5.10 | 8.86 |
| Found | 60.77 | 5.07 | 8.89 |

### EXPERIMENTAL EXAMPLE

Primary cortical cell cultures from embryonic mice were prepared and used to examine neuroprotective action of compounds. Mouse cortical cell culture system has been extensively used to study mechanisms and pharmacological intervention of neuronal death in neurological diseases. In brief, mouse cerebral cortices were removed from brains of the 15 day-old-fetal mice, in accordance with a protocol approved by our institutional animal care committee. The neocortices were gently triturated and plated on 24 well plates (5 hemispheres/plate) precoated with 100 µg/ml poly-D-lysine and 4 µg/ml laminine. Plating media consist of Eagles minimal essential media (MEM, Earles salts, supplied glutamine-free) supplemented with 5% horse serum, 5% fetal bovine serum, 2 mM glutamine, and 21 mM glucose. Cultures were maintained at 37°C in a humidified 5% CO₂ atmosphere. After 7 days in vitro (DIV 7), cultures were shifted into a growth medium identical to the plating medium but lacking fetal serum. At DIV 7 - 9, 10 mM cytosine arabinofuranoside was included to halt overgrowth of glia. Mixed cultures of neurons and glia were then fed twice a week.

To induce neuronal injury by NMDA or Zn²⁺, cortical cell cultures were exposed to toxic doses of NMDA for 10 min or Zn²⁺ for 30 min in a HEPES-buffered control salt solution (HCSS) : (in mM) 120 NaCl, 5KCl, 1.6 MgCl₂, 2.3 CaCl₂, 15 glucose, 20 HEPES and 10 NaOH. After exposure, cultures were washed out 3 times and exchanged with MEM adjusted to 25 mM glucose and 26.2 mM sodium bicarbonate, and placed in the CO₂ incubator for. the next 20 - 24 hr. To induce free radical neurotoxicity, cortical cell cultures were continuously exposed to Fe²⁺ or BSO for 20 - 24 hr, in MEM adjusted to 25 mM glucose and 26.2 mM sodium bicarbonate.

Overall cell injury was assessed microscopically under phase-contrast optics or by measuring amount of lactate dehydrogenase (LDH) released into the bathing medium 24 hr after neurotoxic insults as previously described (Koh and Choi, J Neurosci Methods 20:83-90, 1987). The percent neuronal death was normalized to the mean LDH value released 24 hr after continous exposure 500 µM NMDA (=100) or a sham control (=0).

To examine anti-oxidant effect, DPPH (2,2-diphenyl-1-picryl-hydrazyl radical), a stable free radical, was dissolved in ethanol to make a 100 µM solution. A compound was reacted with DPPH or ethanol. After incubation for 30 min, relative decrease in DPPH absorption at 517 nm was measured by a spectrophotometer.

### Experimental Example 1 : Anti-oxidant action of 5 - amino salicylic acid

The neuroprotective action of 5-amino salicylic acid (AS) was examined in primary cortical cell cultures. Inclusion of 10 - 300 µM AS did not attenuate neuronal death evolving 24 hr after 10 min-exposure to 300 µM NMDA (Fig.1a). Interestingly, addition of 10 - 100 µM AS dose-dependently prevented free radical neurotoxicity following 24 hr-exposure to Fe²⁺ (Fig. 1b). Neuronal death 24 hr after 30 min-exposure to 300 µM Zn²⁺ was not reduced by continuous inclusion of AS during and post Zn²⁺ treatment (Fig. 1c). The neuroprotective action of AS against free radical neurotoxicity was attributable to direct anti-oxidant property of the compound as AS decreased levels of DPPH, a stable free radical (Table 1). Compared to trolox, a membrane-permeable form of vitamin E, AS was a weak anti-oxidant

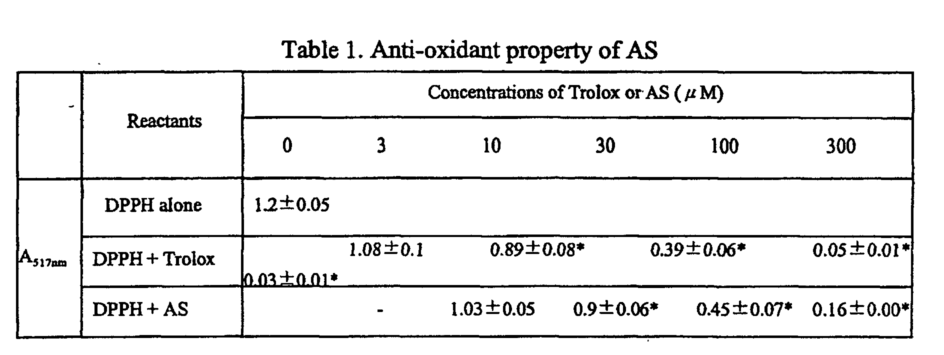

AS or trolox was reacted with 100 uM DPPH dissolved in ethanol for 30 min. Anti-oxidant property was analyzed by measuring changes in the level of DPPH at 517mn, mean ± SEM (n = 3 test tubes per condition), after subtracting background value resulting from ethanol alone. * indicates significant difference from DPPH alone at P < 0.05, using ANOVA and Student-Neuman-Keuls test.

AS can protect neurons from free radical injuries without beneficial effects against NMDA or Zn²⁺ neurotoxicity. However, AS is weaker than trolox in scavenging free radicals.

### Experimental Example 2 : Neuroprotective effects of 5-benzylaminosalicylic acid and its derivatives.

### 1. Neuroprotective effects of 5-benzylaminosalicylic acid

BAS was synthesized and examined against neuronal injuries induced in cortical cell cultures. Concurrent addition of 300 µM 5-benzylaminosalicylic acid (BAS) reduced NMDA-induced neuronal death approximately by 50% (Fig. 2a). Neuronal death following exposure to 50 µM Fe²⁺ (Fig. 2b) or 10 mM BSO (Fig. 2c) was substantially reduced in the presence of 1 µM BAS and near completely blocked by addition of 3 µM BAS. Administration of 30 - 300 µM BAS dose-dependently reduced neuronal death 24 hr following exposure to 300 µM Zn²⁺ for 30 min (Fig 2d). Like AS or trolox, BAS acted as a direct anti-oxidant (Table 2). The anti-oxidant property of BAS was observed at a dose as low as 1 µM.

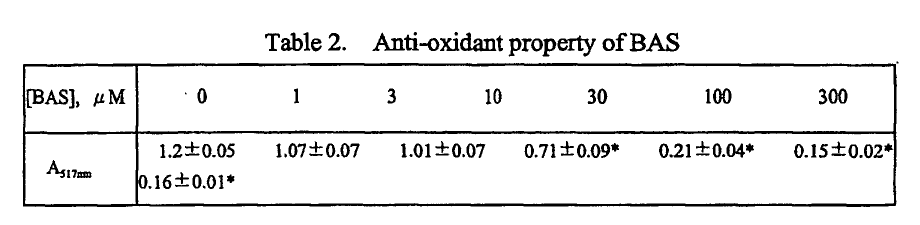

BAS was reacted with 100 uM DPPH dissolved in ethanol for 30 min. Anti-oxidant property was analyzed by measuring changes in DPPH at 517nm, mean ± SEM (n = 3 test tubes per condition), after subtracting background value resulting from ethanol alone. * indicates significant difference from DPPH alone ([BAS] = O) at *P* < 0.05, using ANOVA and Student-Neuman-Keuls test.

### 2. BAS derivatives

BAS derivatives were synthesized by substituting -H at para position of benzylamino group with -NO₂ [5-(4-nitrobenzylamino)salicylic acid, NBAS], -Cl [5-(4-chlorobenzylamino)salicylic acid, CBAS], -CF₃ [5-(4-trifluoromethylbenzylamino)salicylic acid, TBAS]. This substitution with electron-withdrawing group did not reduce neuroprotective effects of BAS against NMDA, Zn²⁺, or free radical neurotoxicity (Figs. 3 - 5). These BAS derivatives were more potent than BAS in preventing free radical neurotoxicity.

**Table 3.**

| Anti-oxidant property ofNBAS | | | | | |
|---|---|---|---|---|---|
| [NBAS],µM | 0 | 10 | 30 | 100 | 300 |
| A₅₁₇ₙₘ | 1.2±0.01 | 0.37±0.1* | 0.04 ± 0.01 * | 0.04±0.00* | 0.04±0.00* |

NBAS was reacted with 100 uM DPPH dissolved in ethanol for 30 min. Anti-oxidant property was analyzed by measuring changes in DPPH at 517nm, mean ± SEM (n = 3 test tubes per condition), after subtracting background value resulting from ethanol alone. * indicates significant difference from DPPH alone ([reactant] = O) at *P* < 0.05, using ANOVA and Student-Neuman-Keuls test.

Substituting -NO₂ with -F or -OCH₃ resulted in decreased neuroprotection against NMDA toxicity but appeared to increase neuroprotective potential against free radical injury (Figs. 6 and 7).

### Experimental Example 3 : Neuroprotective effects of 5-(Pentafluorobenzyl)amino salicylic acid

5-(pentafluorobenzyl)amino salicylic acid (PBAS) was synthesized and tested against neuronal injuries. Concurrent addition of 100 - 1000 µM PBAS reduced NMDA-induced neuronal death in a dose-dependent manner. Treatment with 300 µM PBAS reduced NMDA neurotoxicity approximately by 65% (Fig. 8a). Increasing doses of PBAS up to 1 mM completely blocked neuronal death following exposure to 300 µM NMDA. Inclusion of 1 µM PBAS significantly reduced neuronal death following continuous exposure to 50 µM Fe²⁺. Fe²⁺-induced neuronal death was near completely blocked in the presence of 3 µM PBAS. Free radical neurotoxicity resulting from exposure to 10 mM BSO was blocked by addition of 1 µM PBAS. PBAS reduced levels of DPPH (table 4), suggesting that PBAS blocked free radical neurotoxicity as a direct antioxidant. Concurrent addition of 100 - 300 µM PBAS attenuated Zn²⁺ neurotoxicity (Fig. 8d).

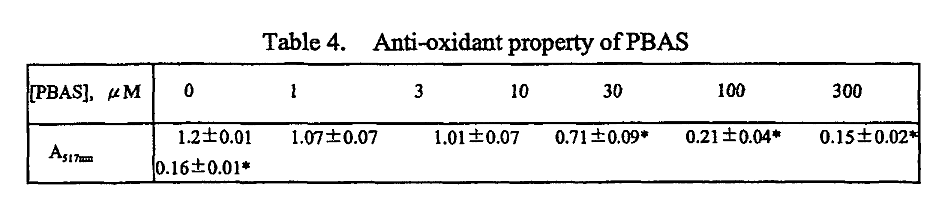

PBAS was reacted with 100 uM DPPH dissolved in ethanol for 30 min. Anti-oxidant property was analyzed by measuring changes in DPPH at 517nm, mean ± SEM (n = 3 test tubes per condition), after subtracting background value resulting from ethanol alone. * indicates significant difference from DPPH alone ([PBAS] = 0) at P < 0.05, using ANOVA and Student-Neuman-Keuls test.

### Experimental Example 4 : Neuroprotective effects ofNBAS derivatives (X = CH₂)

Several derivatives of PBAS such as 5-(4-nitrobenzyl)amino-2-hydroxy ethylbenzoate (NAHE; R₁ = H, R₂ = CH₂CH₃, R₃ = H), 5-(4-nitrobenzyl)-*N*-acetylamino-2-hydroxy ethylbenzoate (NNAHE; R₁ = COCH₃, R₂ = CH₂CH₃, R₃ = H), and 5-(4-nitrobenzyl)-*N*-acetylamino-2-acetoxy ethylbenzoate (NNAAE; R₁ = COCH₃, R₂ = CH₂CH₃, R₃ = COCH₃) were synthesized and their neuroprotection action was examined in cortical cell cultures. These derivatives attenuated NMDA neurotoxicity at 300 µM (Figs. 9 - 11). Inclusion of 10 µM NAHE, NNAHE, or NNAAE blocked Fe²⁺-induced free radical neurotoxicity.

### Experimental Example 5 : Neuroprotective effects of NBAS derivatives (X = CO, SO₂, CH₂CH₂ or CH₂CH₂CH₂)

The group of X (CH₂) of NBAS was substituted with CO [5-(4-nitrobenzoyl) aminosalicylic acid; NBAA], SO₂ [5-(4-nitrobenzenesulfonyl)aminosalicylic acid; NBSAA], CH₂CH₂ [5-(4-nitrophenethyl)aminosalicylic acid; NPAA], or CH₂CH₂CH₂ [5-[3-(4-nitrophenyl)-n-propyl]aminosalicylic acid; NPPAA]. NBAA at a dose of 30 µM significantly attenuated NMDA neurotoxicity. However, its protective effect against NMDA was not further increased up to doses of 300 µM (Fig. 12a). Interestingly, inclusion of 30 - 300 µM NBSAA attenuated NMDA neurotoxicity in a dose-dependent manner (Fig. 13a). With inclusion of 300 µM NBSAA, 90 - 100 % neuronal death following exposure to 300 µM NMDA was markedly reduced. NBAA and NBSAA were still neuroprotective against Fe²⁺ injury but weaker than NBAS in reducing free radical neurotoxicity (Figs. 12 and 13; Table 5).
Substitution of CH₂ for X with CH₂CH₂ or CH₂CH₂CH₂ reduced protective potency of NBAS against NMDA neurotoxicity. Administration of 300 µM NPAA or NPPAA slightly reduced NMDA-induced neuronal death in cortical neurons (Figs. 14 and 15).

In contrast, NPAA or NPPAA turned out to be more effective than NBAS in blocking free radical neurotoxicity as shown by complete blockade of Fe²⁺-induced neuronal death in the presence of 1 µM NPAA or NPPAA.

**Table 5.**

| Anti-oxidant property ofNBAS derivatives | | | | | |
|---|---|---|---|---|---|
| Reactants | DPPH alone | DPPH+NBAA | DPPH+NBSAA | DPPH+NPAA | DPPH+NPPAA |
| A₅₁₇ₙₘ | 1.2 ± 0.01 | 0.42 ± 0.22* | 0.15 ± 0.05* | 0.09 ± 0.01* | 0.09 ± 0.02* |

NBAS derivatives (100 µM for each) were reacted with 100 µM DPPH dissolved in ethanol for 30 min. Anti-oxidant property was analyzed by measuring changes in DPPH at 517nm, mean ± SEM (n = 3 test tubes per condition), after subtracting background value resulting from ethanol alone. * indicates significant difference from DPPH alone at *P* < 0.05, using ANOVA and Student-Neuman-Keuls test.

The above results show that the compounds of Formula (I) may be employed efficiently to prevent neurodegenerative diseases in association with excitotoxicity, Zn²⁺ neurotoxicity and free radical neurotoxicity.

Administration of compounds within Formula (I) to humans can be by any technique capable of introducing the compounds into the bloodstream of a human patient or mammals, including oral administration, and by intravenous, intramuscular and subcutaneous injections The mammals include, but not limited to, cats, dogs, poultry, cattle and the like.

Compounds indicated for prophylactic therapy will preferably be administered in a daily dose generally in a range from about 0.1 mg to about 20 mg per kilogram of body weight per day. A more preferred dosage will be a range from about 0.2 mg to about 10 mg per kilogram of body weight. Most preferred is a dosage in a range from about 0.5 to about 5 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The active compound is usually administered in a pharmaceutically-acceptable formulation. Such formulations may comprise the active compound together with one or more pharmaceutically-acceptable carriers or diluents. Other therapeutic agents may also be present in the formulation. A pharmaceutically-acceptable carrier or diluent provides an appropriate vehicle for delivery of the active compound without introducing undesirable side effects. Delivery of the active compound in such formulations may be performed by various routes including oral, nasal, topical, buccal and sublingual, or by parenteral administration such as subcutaneous, intramuscular, intravenous and intradermal routes.

Formulations for oral administration may be in the form of capsules containing the active compound dispersed in a binder such as gelatin or hydroxypropylmethyl cellulose, together with one or more of a lubricant, preservative, surface-active or dispersing agent. Such capsules or tablets may contain controlled-release formulation as may be provided in a disposition of active compound in hydroxypropylmethyl cellulose.

Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. REFERENCES
A. I. Faden. *Pharmacol.Toxicol.* 78 (1):12-17,1996.
C. K. Joo, J. S. Choi, H. W. Ko, K. Park, S. Sohn, M. H. Chun, Y. J. Oh and B. J. Gwag. IOVS (16) 43:22, 1999.
D. E. Bredesen, M. Wiedau-Pazos, J. J. Goto, S. Rabizadeh, J. A. Roe, E. B. Gralla, L. M. Ellerby, and J. S. Valentine. *Neurology* 47:S36-8,1996.
J.W. Olney. *Retina* 2:341-359, 1982.
D. W. Choi and J. Y. Koh. *Annu.Rev.Neurosci.* 21:347-75:347-375, 1998.
D. W. Choi. *Neuron* 1 :623-634, 1988.
E. D. Hall, J. M. Braughler, and J. M. McCall. *J.Neurotrauma.* 5 (1):81-89,1988.
E. Y. Kim, J. Y. Koh, Y. H. Kim, S. Sohn and B. J. Gwag. Eur. J Neurosci. 11: 327-334. 1999
J. L. Montastruc, O. Rascol, and J. M. Senard. *Neurosci.Biobehav.Rev.* 21 (4):477-480, 1997.
K. A. Jellinger and C. Bancher. *J.Neural Transm.Suppl.* 54:77-95:77-95, 1998.
M. F. Beal. *Ann.Neurol.* 38 (3):357-366, 1995.
M. P. Cuajungco and G. J. Lees. *Brain Res.Rev.* 23 (3):219-236, 1997.
P. H. Chan. *Stroke* 27 (6):1124-1129, 1996.
S. J. Won, E. C. Park, B. R. Ryu, H. W. Ko, S. Sohn, H. J. Kwon, and B. J. Gwag. *Neurobiology of Disease* (in press), 2000

## Claims

1. A compound represented by the following formula : 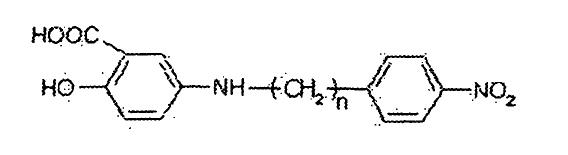 wherein, n is an integer of 2 or 3.

2. A pharmaceutical composition for protecting central neurons from acute or chronic injuries to central nervous system(CNS) comprising one or more of compounds represented by the following formula (I) or pharmaceutically acceptable salts thereof as an effective component: 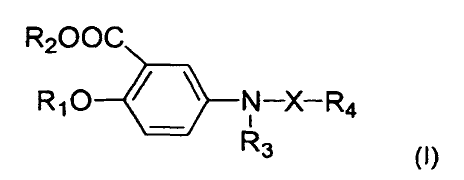 wherein,
X is CO, SO₂ or (CH₂)ₙ (where n is an integer of 1 to 5, indusive);
R₁ is hydrogen, alkyl or alkanoyl;
R₂ is hydrogen or alkyl;
R₃ is hydrogen or an acetoxy group; and
R₄ is phenyl group which is unsubstituted or substituted with one or more of the group consisting of nitro, halogen, haloalkyl, and C₁- C₅ alkoxy; or a pharmaceutically-acceptable salt thereof.

3. The pharmaceutical composition according to Claim 2, wherein said CNS injuries result from ischemia, hypoxia, hypoglycemia, traumatic brain injury, traumatic spinal cord injury, epilepsy, Huntington's disease, Parkinson's disease, Alzheimer's disease, or Amytrophic lateral sclerosis.

4. The pharmaceutical composition according to Claim 2, wherein said CNS injuries are caused by activation of N-methyl-D-aspartate (NMDA) glutamate receptors, entry and accumulation of Zn²⁺, or free radicals.

5. The pharmaceutical composition according to any one of Claim 2 to Claim 4, wherein said composition attenuates NMDA neurotoxicity, Zn²⁺ neurotoxicity, and blocks free radical neurotoxicity as a direct antioxidant.

6. The pharmaceutical composition according to Claim 2, wherein the compounds are selected from the group consisting of
5-benzylaminosalicylic acid (BAS),
5-(4-nitrobenzyl)aminosalicylic acid (NBAS),
5-(4-chlorobenzyl)aminosalicylic acid (CBAS),
5-(4-trifluoromethylbenzyl)aminosalicylic acid (TBAS),
5-(4-fluorobenzyl)aminosaticylic acid (FBAS),
5-(4-methoxybenzyl)aminosalicylic acid (MBAS)
5-(4-pentafluorobenzyl)aminosalicylic acid (PBAS),
5-(4-nitrobenzyl)amino-2-hydroxy ethylbenzoate,
5-(4-nitrobenzyl)-*N*-acetylamino-2-hydroxy ethylbenzoate,
5-(4-nitrobenzyl)-*N*-acetylamino-2-acetoxy ethylbenzoate,
5-(4-nitrobenzoyl)aminosalicylic acid,
5-(4-nitrobenzenesulfonyl)aminosalicylic acid,
5-[2-(4-nitrophenyl)ethyl]aminosalicylic acid, and
5-[3-(4-nitrophenyl)-n-propyl]aminosalicylic acid, or a pharmaceutically-acceptable salt thereof.

## Patentansprüche

1. Eine Verbindung, die durch die folgende Formel dargestellt wird: 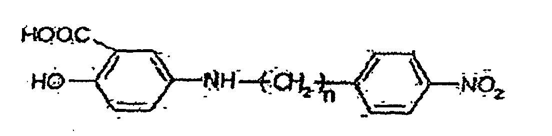 worin n eine ganze Zahl von 2 oder 3 ist.

2. Eine pharmazeutische Zusammensetzung zum Schutz zentraler Neuronen vor akuten oder chronischen Verletzungen des zentralen Nervensystems (ZNS), die eine oder mehrere der Verbindungen, die durch die folgende Formel (I) dargestellt werden oder pharmazeutisch verträgliche Salze davon als eine wirksame Komponente umfasst: 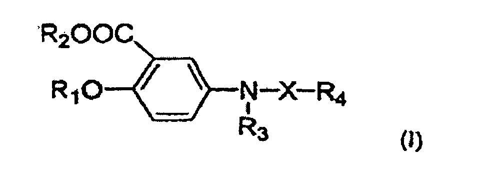 worin
X CO, SO₂ oder (CH₂)ₙ ist (worin n eine ganze Zahl von 1 bis 5 ist, einschließlich);
R₁ ist Wasserstoff, Alkyl oder Alkanoyl;
R₂ ist Wasserstoff oder Alkyl;
R₃ ist Wasserstoff oder eine Acetoxygruppe; und
R₄ ist eine Phenylgruppe, die nicht subsituiert ist oder mit einer oder mehreren der Gruppe substituiert ist, die aus Nitro, Halogen, Haloalkyl und C₁- C₅ -Alkoxy besteht; oder ein pharmazeutisch verträgliches Salz davon.

3. Die pharmazeutische Zusammensetzung gemäß Anspruch 2, worin besagte ZNS-Verletzungen aus Ischämie, Hypoxie, Hypoglykämie, traumatischer Gehirnverletzung, traumatischer Rückenmarksverletzung, Epilepsie, Huntington's Erkrankung, Parkinson's Erkrankung, Alzheimer's Erkrankung oder amytropher lateraler Sklerose resultieren.

4. Die pharmazeutische Zusammensetzung gemäß Anspruch 2, worin besagte ZNS-Verletzungen durch Aktivierung der N-Methyl-D-Aspartat (NMDA)-Glutamatrezeptoren, Einstrom und Anhäufung von Zn²⁺ oder freie Radikale bewirkt werden.

5. Die pharmazeutische Zusammensetzung gemäß einem der Ansprüche 2 bis 4, worin besagte Zusammensetzung NMDA-Neurotoxizität, Zn²⁺-Neurotoxizität vermindert und freie Radikalneurotoxizität als ein direktes Antioxidanz blockiert.

6. Die pharmazeutische Zusammensetzung gemäß Anspruch 2, worin die Verbindungen aus der Gruppe ausgewählt sind, die aus
5-Benzylaminosalicylsäure (BAS),
5-(4-Nitrobenzyl)aminosalicylsäure (NBAS),
5-(4-Chlorbenzyl)aminosalicylsäure (CBAS),
5-(4-Trifluormethylbenzyl)aminosalicylsäure (TBAS),
5-(4-Fluorbenzyl)aminosalicylsäure (FBAS),
5-(4-Methoxybenzyl)aminosalicylsäure (MBAS),
5-(4-Pentafluorbenzyl)aminosalicylsäure (PBAS),
5-(4-Nitrobenzyl)amino-2-hydroxyethylbenzoat,
5-(4-N itrobenzyl)-N-acetylamino-2-hydroxyethylbenzoat,
5-(4-Nitrobenzyl)-*N*-azetylamino-2-acetoxybenzoat,
5-(4-Nitrobenzyl)aminosalicylsäure,
5-(4-Nitrobenzolsulfonyl)aminosalicylsäure,
5-[2-(4-Nitrophenyl)ethyl]aminosalicylsäure, und
5-[3-(4-Nitrophenyl)-n-propyl]aminosalicylsäure oder einem pharmazeutisch verträglichen Salz davon, besteht.

## Revendications

1. Composé représenté par la formule suivante : 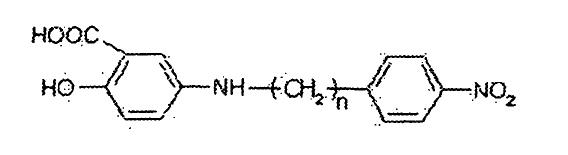 où n est un entier de 2 ou 3.

2. Composition pharmaceutique pour la protection de neurones centraux contre des lésions aiguës ou chroniques du système nerveux central (SCN), comprenant un ou plusieurs composés représentés par la formule (I) suivante ou leurs sels acceptables du point de vue pharmaceutique en tant qu'un constituant efficace : 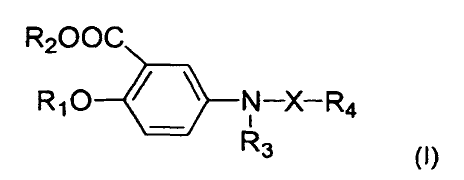 où
X représente CO, SO₂ ou (CH₂)ₙ (où n est un entier de 1 à 5 compris) ;
R₁ représente un atome d'hydrogène, un groupe alkyle ou alcanoyle ;
R₂ représente un atome d'hydrogène ou un groupe alkyle ;
R₃ représente un atome d'hydrogène ou un groupe acétoxy ; et
R₄ représente un groupe phényle, qui est non substitué ou substitué par un ou plusieurs groupes parmi le groupe consistant en nitro, halogène, haloalkyle et alkoxy en C₁-C₅ ; ou est un sel de celui-ci acceptable du point de vue pharmaceutique.

3. Composition pharmaceutique selon la revendication 2, où lesdites lésions du SNC sont provoquées par l'ischémie, l'hypoxie, l'hypoglycémie, un traumatisme crânien, des lésions traumatique de la moëlle épinière, l'épilepsie, la maladie de Huntington, la maladie de Parkinson, la maladie d'Alzheimer ou la sclérose latérale amyotrophique.

4. Composition pharmaceutique selon la revendication 2, où lesdites lésions du SNC sont provoquées par l'activation des récepteurs N-méthyl-D-aspartate (NMDA) glutamate, par l'entrée et l'accumulation de Zn²⁺, ou de radicaux libres.

5. Composition pharmaceutique selon l'une quelconque des revendications 2 à 4, où ladite composition atténue la neurotoxicité du NMDA, la neurotoxicité de Zn²⁺ et bloque la neurotoxicité des radicaux libres en tant qu'anti-oxydant direct.

6. Composition pharmaceutique selon la revendication 2, où les composés sont pris dans le groupe consistant en :
acide 5-benzylaminosalicylique (BAS),
acide 5-(4-nitrobenzyl)aminosalicylique (NBAS),
acide 5-(4-chlorobenzyl)aminosalicylique (CBAS),
acide 5-(4-trifluorométhylbenzyl)aminosalicylique (TBAS),
acide 5-(4-fluorobenzyl)aminosalicylique (FBAS),
acide 5-(4-méthoxybenzyl)aminosalicylique (MBAS)
acide 5-(4-pentafluorobenzyl)aminosalicylique (PBAS),
benzoate de 5-(4-nitrobenzyl)amino-2-hydroxyéthyle,
benzoate de 5-(4-nitrobenzyl)-N-acétylamino-2-hydroxyéthyle,
benzoate de 5-(4-nitrobenzyl)-N-acétylamino-2-acétoxyéthyle,
acide 5-(4-nitrobenzoyl)aminosalicylique,
acide 5-(4-nitrobenzènesulfonyl)aminosalicylique,
acide 5-[2-(4-nitrophényl)éthyl]aminosalicylique et
acide 5-[3-(4-nitrophényl)-n-propyl]aminosalicylique
ou un de leurs sels acceptables en pharmacie.
